(19) Europäisches Patentamt
European Patent Office
Office européen des brevets

(11) Veröffentlichungsnummer : **0 084 155**
**B1**

(12) # EUROPÄISCHE PATENTSCHRIFT

(45) Veröffentlichungstag der Patentschrift : **25.03.87**

(21) Anmeldenummer : **82111881.7**

(22) Anmeldetag : **21.12.82**

(51) Int. Cl.⁴ : **C 07 D498/04**, C 07 D487/04, C 07 D513/04, C 07 D409/04, C 07 D405/04, C 07 D403/04, A 61 K 31/55 // (C07D498/04, 265:00, 243:00),(C07D487/04, 243:00, 241:00)

(54) **(1,2)-anellierte 1,4-Benzodiazepin-Verbindungen sowie Verfahren zu ihrer Herstellung und diese Verbindungen enthaltende Arzneimittel.**

(30) Priorität : **28.12.81 DE 3151597**

(43) Veröffentlichungstag der Anmeldung : **27.07.83 Patentblatt 83/30**

(45) Bekanntmachung des Hinweises auf die Patenterteilung : **25.03.87 Patentblatt 87/13**

(84) Benannte Vertragsstaaten : **AT BE CH DE FR GB IT LI LU NL SE**

(56) Entgegenhaltungen :
**EP-A- 0 008 045**
**DE-A- 2 314 993**
**US-A- 3 403 161**

(73) Patentinhaber : **Kali-Chemie Pharma GmbH**
**Hans-Böckler-Allee 20 Postfach 220**
**D-3000 Hannover 1 (DE)**

(72) Erfinder : **Liepmann, Hans Dipl.-Chem. Dr. rer. nat.**
**Auf dem Emmerberg 17**
**D-3000 Hannover 1 (DE)**
Erfinder : **Ruhland, Michael Dipl.-Biol. Dr. rer. nat.**
**Bachstelzenweg 14**
**D-3000 Hannover 61 (DE)**
Erfinder : **Müsch, Herbert Dipl.-Biol. Dr.**
**Am Schönen Hoope 16**
**D-3015 Wennigsen 5 (DE)**
Erfinder : **Benson, Werner Dipl.-Chem. Dr. rer. nat.**
**In der Steinbreite 39**
**D-3000 Hannover 91 (DE)**
Erfinder : **Heinemann, Henning Dipl.-Chem. dr. rer. nat.**
**Bergiusstrasse 18**
**D-3000 Hannover 51 (DE)**
Erfinder : **Zeugner, Horst Dipl.-Chem. Dr. rer. nat.**
**Havelweg 10**
**D-3000 Hannover 73 (DE)**

(74) Vertreter : **Lauer, Dieter, Dr.**
**c/o Kali-Chemie AG Postfach 220 Hans-Böckler-Allee 20**
**D-3000 Hannover 1 (DE)**

**EP 0 084 155 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue [1,2]-anellierte 7-Heteroaryl-1,4-benzodiazepin-Verbindungen und deren Salze sowie diese Verbindungen enthaltende pharmazeutische Zubereitungen und Verfahren zur Herstellung dieser Verbindungen.

Aus US-A-3 403 161 sind in 2-Stellung unsubstituierte 5-Pyridyl-2,3-dihydro-1H-1,4-benzodiazepine mit sedativen, anticonvulsiven und muskelrelaxierenden Eigenschaften bekannt. In DE-A-2 314 993 werden in 2-Stellung eine substituierte Methylgruppe tragende und in 5-Stellung durch Thienyl oder Furyl substituierte 2,3-Dihydro-1H-1,4-benzodiazepine beschrieben und für diese ebenfalls sedative und anticonvulsive Eigenschaften angegeben.

Aus der EP-A-0 008 045 und der dieser entsprechenden DE-A-2 835 708 sind 5-Phenyl-2,3-dihydro-1,4-benzodiazepinderivate bekannt, an deren Grundgerüst in 1,2-Stellung ein weiterer heterocyclischer Ring anelliert ist. Diese [1,2]-anellierten 7-Phenyl-1,4-benzodiazepinderivate besitzen, ausgeprägte ulcushemmende Wirkung und zeigen dabei nur eine verhältnismäßig geringe ZNS-Wirksamkeit.

Der Erfindung liegt die Aufgabe zugrunde, neue [1,2]-anellierte 1,4-Benzodiazepin-Verbindungen mit wertvollen pharmakologischen Eigenschaften zu entwickeln.

Die erfindungsgemäßen [1,2]-anellierten 7-Heteroaryl-1,4-benzodiazepin-Verbindungen unterscheiden sich von den vorbekannten 5-Heteroaryl-1,4-benzodiazepinen des US-Patentes 3 403 161 und der DE-A-2 314 993 in ihrer chemischen Struktur wesentlich durch das Vorhandensein eines weiteren, in 1,2-Stellung anellierten Heterocyclus. Von den [1,2]-anellierten 7-Phenyl-1,4-benzodiazepinen der EP-A-0 008 045 unterscheiden sich die erfindungsgemäßen Verbindungen strukturell dadurch, daß sie eine 7-Heteroarylgruppe anstelle der 7-Phenylgruppe enthalten.

Es wurde nun gefunden, daß die erfindungsgemäßen [1,2]-anellierten 7-Heteroaryl-1,4-benzodiazepin-Verbindungen ein neuartiges pharmakologisches Wirkungsprofil mit ausgeprägter neuroleptischer Wirkungskomponente bei guter therapeutischer Breite und geringer Toxizität aufweisen.

Die vorliegende Erfindung betrifft daher [1,2]-anellierte 1,4-Benzodiazepin-Verbindungen der allgemeinen Formel I

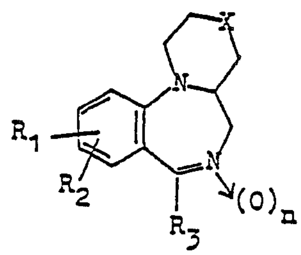

(I)

worin

X für Sauerstoff, Schwefel oder eine Iminogruppe =N—$R_4$ steht, worin

$R_4$ Wasserstoff, $C_1$-$C_5$-Alkyl, endständig durch Methoxy oder Hydroxy substituiertes $C_2$-$C_5$-Alkyl, $C_3$-$C_5$-Alkenyl oder Cyclopropylmethyl bedeutet ;

$R_1$ Wasserstoff, Alkyl mit 1-4 Kohlenstoffatomen, Halogen, Alkoxy mit 1-4 Kohlenstoffatomen oder Nitro bedeutet, und

$R_2$ Wasserstoff, Alkyl mit 1-4 Kohlenstoffatomen, Halogen, Alkoxy mit 1-4 Kohlenstoffatomen oder Nitro bedeutet, oder, falls $R_1$ Wasserstoff ist, auch Alkylthio mit 1-4 Kohlenstoffatomen oder, sofern außerdem X S oder eine =N—$R_4$ Gruppe ist, auch Trifluormethyl bedeutet ;

oder

$R_1$ und $R_2$ an benachbarte Kohlenstoffatome gebunden sind und zusammen Methylendioxy oder Äthylendioxy bedeuten ;

$R_3$ für eine der folgenden Gruppen a, b oder c steht

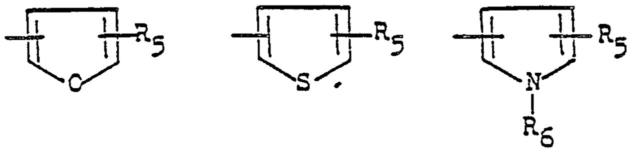

a b c

worin

$R_5$ Wasserstoff, Alkyl mit 1-4 Kohlenstoffatomen, Fluor, Chlor, Brom oder Nitro bedeutet, und

$R_6$ Wasserstoff, $C_1$-$C_5$-Alkyl, endständig durch Hydroxy oder Methoxy substituiertes $C_2$-$C_5$-Alkyl, $C_3$-$C_5$-Alkenyl oder Cyclopropylmethyl bedeutet ; und

n 0 oder, falls $R_3$ für die Gruppe a oder b steht, auch 1 bedeutet; und deren optische Isomere und Säureadditionssalze.

Sofern in den Verbindungen der Formel I die Substituenten $R_1$ und $R_2$ eine niedere Alkyl-Gruppe darstellen oder enthalten, bedeutet diese eine gerade oder verzweigte Alkyl-Gruppe mit 1-4 Kohlenstoffatomen, insbesondere Methyl oder Äthyl. So stellen diese niederen Alkyl-Substituenten bevorzugt Methyl und die niederen Alkoxy- oder Alkylthiosubstituenten bevorzugt Methoxy oder Methylthio dar. Für die Substituenten $R_1$ und $R_2$ kommen als Halogenatome insbesondere Fluor, Chlor oder Brom in Frage. Die Substituenten $R_1$ und $R_2$ sind bevorzugt in 9- und/oder 10-Stellung oder, falls sie $NO_2$ oder $CF_3$ bedeuten, in 9-Stellung angeordnet und stellen vorzugsweise Wasserstoff, Methoxy, Methyl, Chlor, Brom oder Fluor dar.

Falls X für eine =N—$R_4$-Gruppe steht und $R_4$ eine, wie vorstehend definiert gegebenenfalls substituierte Alkyl-Gruppe bedeutet, stellt diese vorzugsweise eine geradkettige Alkyl-Gruppe mit 1-5, insbesondere 1-3 Kohlenstoffatomen dar. Vorzugsweise steht $R_4$ für Methyl, Äthyl, Methoxyäthyl oder Hydroxyäthyl.

Falls $R_5$ niederes Alkyl bedeutet, ist dieser Restgerade oder verzweigt, enthält 1-4 Kohlenstoffatome und stellt vorzugsweise Methyl oder Äthyl dar.

Sofern $R_3$ für eine Furyl-Gruppe a steht, bedeutet $R_5$ insbesondere Wasserstoff, $C_1$-$C_4$-Alkyl oder Nitro, bevorzugt Wasserstoff oder Methyl.

Falls $R_3$ eine Thienyl-Gruppe b darstellt, steht $R_5$ insbesondere für Wasserstoff, niederes Alkyl, bevorzugt Methyl, Fluor, Chlor, Brom oder auch Nitro.

Falls $R_3$ für eine Pyrrolyl-Gruppe c steht, bedeutet $R_5$ insbesondere Wasserstoff oder Methyl. Falls $R_6$ eine gegebenenfalls substituierte Alkyl-Gruppe wie oben definiert, darstellt, ist diese vorzugsweise geradkettig und enthält 1-5, vorzugsweise 1-3 Kohlenstoffatome und stellt bevorzugt Methyl, Äthyl, Methoxyäthyl oder Hydroxyäthyl dar. Vorzugsweise bedeutet $R_6$ Methyl. Falls X eine =N—$R_4$-Gruppe darstellt, können $R_4$ und $R_6$ vorteilhaft identisch sein.

Erfindungsgemäß werden die neuen [1,2]-anellierten-Benzodiazepin-Verbindungen der Formel I sowie deren optische Isomere und Säureadditionssalze erhalten, indem man in an sich bekannter Weise

a) Verbindungen der allgemeinen Formel II oder III

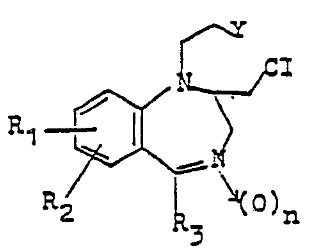

(II)

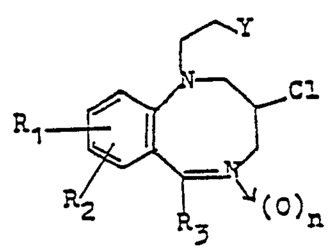

(III)

worin $R_1$, $R_2$, $R_3$ und n obige Bedeutung besitzen und Y Halogen, $C_1$-$C_4$-Alkansulfonyloxy, Benzolsulfonyloxy oder im Benzolring durch $C_1$-$C_4$-Alkyl oder Halogen substituiertes Benzolsulfonyloxy bedeutet, oder deren Gemische mit einem Alkalimetallhydroxyd, einem Alkalimetallsulfid oder einem Amin der Formel $R_4$—$NH_2$, worin $R_4$ obige Bedeutung besitzt, umsetzt, oder

b) zur Herstellung von Verbindungen der Formel Ia

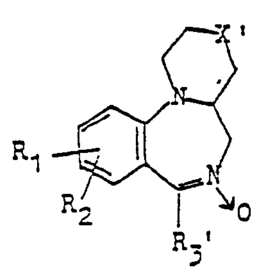

(Ia)

worin $R_1$ und $R_2$ obige Bedeutung besitzen, X' Sauerstoff oder Schwefel bedeutet, und $R_3'$ für eine Gruppe a oder b steht,

Verbindungen der Formel Ib

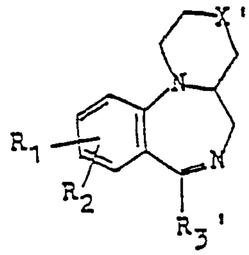

(Ib)

worin $R_1$, $R_2$, $R_3'$ und X' obige Bedeutung besitzen, oxidiert, oder

c) zur Herstellung von Verbindungen der Formel Ic

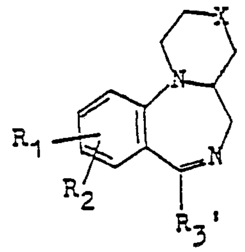

(Ic)

worin $R_1$, $R_2$, $R_3'$ und X obige Bedeutung besitzen, Verbindungen der Formel Id

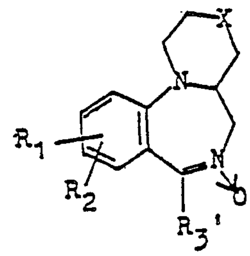

(Id)

worin $R_1$, $R_2$, $R_3'$ und X obige Bedeutung besitzen, reduziert, oder

d) zur Herstellung von Verbindungen der Formel Ie

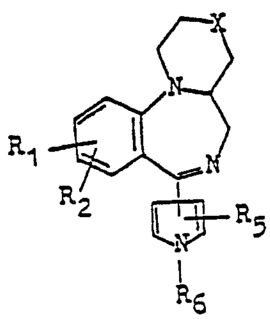

(Ie)

worin $R_1$, $R_2$, $R_5$, $R_6$ und X obige Bedeutung besitzen, Verbindungen der Formel IV

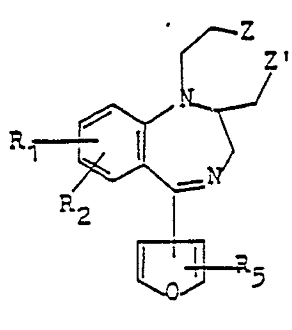

(IV)

worin $R_1$, $R_2$ und $R_5$ obige Bedeutung besitzen, und Z die für Y angegebene Bedeutung besitzt und Z' Chlor bedeutet oder Z und Z' gemeinsam Sauerstoff, Schwefel oder eine Gruppe $=NR_4$ bedeuten, mit

einem Amin der Formel $R_6$—$NH_2$, worin $R_6$ obige Bedeutung besitzt, umsetzt, und gegebenenfalls in Verbindungen der Formel I worin $R_1$ und/oder $R_2$ Wasserstoff bedeuten, in den Phenylring Chlor, Brom oder, falls $R_3$ nicht für die Gruppe c steht, auch Nitro einführt, und/oder gegebenenfalls racemische Gemische von Verbindungen der Formel I in ihre optischen Isomeren auftrennt und/oder gegebenenfalls freie Verbindungen der Formel I in ihre Säureadditionssalze überführt oder die Säureadditionssalze in die freien Verbindungen der Formel I überführt.

Die Cyclisierung gemäß Verfahren a kann unter aus der DE-A-2 835 708 zur Bildung von [1,2]-anellierten Benzodiazepin-Verbindungen bekannten Ringschluß-Bedingungen erfolgen. Als Ausgangsverbindungen können die 2-Chlormethyl-1,4-benzodiazepin-Verbindungen der Formel II aber auch die 3-Chlor-1,5-benzodiazocin-Verbindungen der Formel III oder Gemische von Benzodiazepinen der Formel II mit entsprechenden Benzodiazocinen der Formel III eingesetzt werden, da das Benzodiazocingerüst der Verbindungen der Formel III unter den Cyclisierungsbedingungen in das Benzodiazepingerüst umgewandelt wird. Falls Y in den Verbindungen der Formel II und/oder III Halogen bedeutet, kann dieses Chlor, Brom, Jod, vorzugsweise jedoch Chlor darstellen. Falls Y einen substituierten Benzols sulfonyloxyrest bedeutet, stellt es vorzugsweise eine Toluolsulfonyloxygruppe dar.

Zur Herstellung der anellierten Oxazino- und Thiazino-[4,3-a] [1,4] benzodiazepin-Verbindungen der Formel I (X=O oder S) werden die Verbindungen der Formel II und/oder III mit Alkalimetallhydroxiden oder -sulfiden, beispielsweise mit Natrium- oder Kaliumhydroxid oder -sulfid umgesetzt. Zur Herstellung der anellierten Pyrazino [1,2-a] [1,4] benzodiazepin-Verbindungen der Formel I (X=N—$R_4$) werden die Verbindungen der Formel II und/oder III mit einem Amin der Formel $R_4$—$NH_2$ umgesetzt. Zweckmäßig wird das jeweilige Cyclisierungsreagenz im Überschuß eingesetzt. Die cyclisierende Umsetzung wird zweckmäßig in einem unter den Reaktionsbedingungen inerten Lösungsmittel bei Temperaturen zwischen etwa 50 und 150 °C durchgeführt. Als Lösungsmittel für die Cyclisierung eignen sich insbesondere organische Lösungsmittel, beispielsweise niedere Alkohole wie Methanol, Äthanol oder Isopropanol, niedere Ketone wie Aceton, niedere offene oder cyclische Äther wie Diäthyläther, Dioxan oder Tetrahydrofuran, Pyridin, Dimethylsulfoxid oder Dimethylformamid, wobei die Zugabe von Wasser vorteilhaft sein kann. Sofern als Cyclisierungsmittel ein Amin $R_4$—$NH_2$ eingesetzt wird, kann dieses Amin auch als Lösungsmittel verwendet werden. Bei der Umsetzung von Verbindungen der Formel II und/oder III, worin $R_3$ für eine Furangruppe steht und n 0 bedeutet, mit einem Amin $R_4$—$NH_2$ erfolgt unter den Cyclisierungsbedingungen bei Temperaturen oberhalb 75 °C in dem Rest $R_3$ weitgehend ein Austausch von Sauerstoff gegen die N—$R_4$-Gruppe, so daß als Hauptprodukte Verbindungen der Formel I, worin $R_3$ eine Pyrrolylgruppe darstellt, erhalten werden. Falls die cyclisierende Umsetzung mit dem Amin jedoch bei Temperaturen unterhalb 75 °C oder in Gegenwart von einer starken Base, zum Beispiel einer anorganischen Base wie Natriumhydroxid, durchgeführt wird, unterbleibt ein derartiger Austausch weitgehend, und der Furanring bleibt erhalten.

N-Oxidverbindungen der Formel Ia können auch erhalten werden, indem man gemäß Verfahren b Verbindungen der Formel Ib in an sich bekannter Weise zu ihren entsprechenden N-Oxiden oxidiert. Die Oxidation kann zum Beispiel mit organischen Persäuren in einem unter den Reaktionsbedingungen inerten organischen Lösungsmittel, beispielsweise nach den in Chem. Rev. *68*, 747 (1968) beschriebenen Verfahren, erfolgen. Insbesondere eignen sich als Oxidationsmittel Perbenzoesäuren wie zum Beispiel 3-Chlorperbenzoesäure. Als Lösungsmittel eignen sich zum Beispiel halogenierte Kohlenwasserstoffe wie Methylenchlorid.

Verbindungen der Formel Ic können auch erhalten werden, indem man in an sich bekannter Weise die entsprechenden N-Oxide der Formel Id reduziert (siehe zum Beispiel J. Org. Chem. *26*, 1.111 (1961)). Als Reduktionsmittel eignet sich insbesondere $PCl_3$ in einem unter den Reaktionsbedingungen inerten organischen Lösungsmittel, beispielsweise einem halogenierten Kohlenwasserstoff wie Chloroform oder Methylenchlorid. Die Herstellung von Verbindungen der Formel I durch Reduktion entsprechender N-Oxide ist insbesondere geeignet zur Herstellung von 7-Furyl-pyrazino [1,2-a] [1,4]-benzodiazepin-Verbindungen der Formel I ($R_3$=a, X=$NR_4$). Bei der Reduktion mit $PCl_3$ kann in geringem Maße Chlorierung im Furyl-Ring $R_3$ stattfinden, so daß entsprechende chlorierte Verbindungen der Formel I ($R_5$=Cl) als Nebenprodukte erhalten werden können.

Gemäß Verfahren d können Furyl-Verbindungen der Formel IV durch Umsetzung mit einem Amin $R_6$—$NH_2$ in Pyrrol-Verbindungen der Formel Ie umgewandelt werden. Die Umwandlung des Furylrestes in den Pyrrolylrest kann bei Temperaturen zwischen 80 und 150 °C unter den unter Verfahren a beschriebenen Cyclisierungsbedingungen erfolgen.

Eine nachträgliche Substitution im Phenylring des 1,4-Benzodiazepin-Gerüstes ist sowohl in den anellierten Endprodukten der Formel I wie auch auf der Stufe der cyclisierten Zwischenprodukte der Formeln II und/oder III in an sich bekannter Weise durch Halogen oder, falls $R_3$ nicht Pyrrolyl darstellt, auch die Nitrogruppe möglich. Als Halogenierungsmittel können beispielsweise N-Chlorsuccinimid oder N-Bromsuccinimid dienen. Zur Einführung der Nitrogruppe können die üblichen Nitrierungsreagenzien herangezogen werden, beispielsweise $KNO_3$ in $H_2SO_4$ oder als schonendes Nitrierungsreagenz Kupfer-II-nitrat-trihydrat in Acetanhydrid.

Gewünschtenfalls kann eine vorhandene Nitrogruppe auch nachträglich in an sich bekannter Weise in Halogen überführt oder völlig abgespalten werden.

Die nach den erfindungsgemäßen Verfahren erhaltenen Verbindungen der Formel I können in an sich

bekannter Weise in Form der freien Basen oder ihrer Säureadditionssalze isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden. Dazu versetzt man beispielsweise eine Lösung einer Verbindung der Formel I in einem Lösungsmittel mit der als Salzkomponente gewünschten Säure. Vorzugsweise wählt man für die Umsetzung organische Lösungsmittel, in denen das entsprechende Salz schwer löslich ist. Zur Bildung pharmakologisch akzeptabler Salze von Verbindungen der Formel I eignen sich beispielsweise anorganische Säuren wie Chlorwasserstoffsäure, Schwefelsäure oder Phosphorsäure, oder organische Säuren, beispielsweise organische Sulfonsäuren wie Methan- oder Toluolsulfonsäuren oder Cyclohexylaminosulfonsäure, niedere aliphatische Carbonsäuren, welche gegebenenfalls hydroxyliert sein oder eine Doppelbindung enthalten können, wie Milchsäure, Bernsteinsäure, Weinsäure, Fumarsäure, Maleinsäure oder Citronensäure, oder niedere aromatische Carbonsäuren wie Benzoesäure.

Die erfindungsgemäßen Verbindungen der Formel I werden bei der Synthese in Form ihrer Racemate erhalten. Unter den Schutz dieser Erfindung fallen nun sowohl die racemischen Gemische wie auch die optisch aktiven Formen der Verbindungen der Formel I. Die optisch aktiven Verbindungen können aus den racemischen Gemischen in an sich bekannter Weise durch Salzbildung mit geeigneten optisch aktiven Säuren, wie beispielsweise Weinsäure, und anschließende fraktionierte Kristallisation der gewonnenen Salze in ihre optisch aktiven Antipoden aufgetrennt werden.

Die als Ausgangssubstanzen verwendeten Benzodiazepin-Verbindungen der Formel II und Benzodiazocin-Verbindungen der Formel III sind neu. Ihre Herstellung kann in an sich bekannter Weise erfolgen, beispielsweise nach den in den DE-A-2 221 558, 2 353 187 oder 2 810 349 beschriebenen Methoden.

Insbesondere kann man 2-Hydroxy-1,3-diaminopropane der Formel V

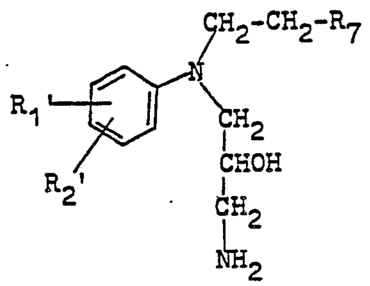

(V)

worin $R_1'$ und $R_2'$ die für $R_1$ und $R_2$ angegebene Bedeutung mit Ausnahme von Nitro besitzen und $R_7$ Hydroxyl oder Methoxy bedeutet, mit einem Acylchlorid der Formel VI

$$R_3'—CO—Cl \qquad VI$$

worin $R_3'$ obige Bedeutung besitzt, zu Acylaminen der Formel VII

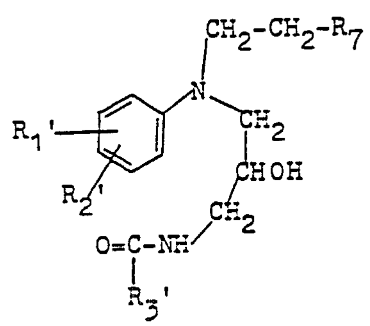

(VII)

worin $R_1'$, $R_2'$, $R_3'$ und $R_7$ obige Bedeutung besitzen, acylieren, und anschließend die Verbindungen der Formel VII in an sich bekannter Weise durch Umsetzung mit Phosphoroxidhalogeniden, vorzugsweise Phosphoroxidchlorid, cyclisieren. Zweckmäßigerweise behandelt man dazu die Verbindungen der Formel VII oder deren Säureadditionssalze wie in der DE-A-2 520 937 beschrieben mit Phosphoroxidchlorid bei einer Temperatur zwischen 100 und 150 °C, vorzugsweise bei Siedetemperatur des Reaktionsgemisches. Hierbei wird ein Gemisch der beiden isomeren Verbindungen der Formeln VIII und IX

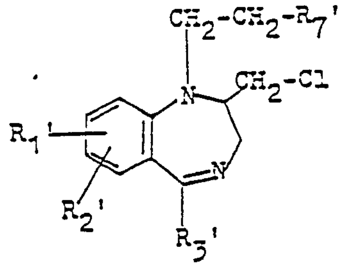

(VIII)

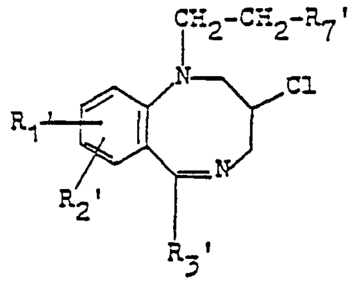

(IX)

worin $R_1'$, $R_2'$ und $R_3'$ obige Bedeutung besitzen und $R_7'$ Chlor oder Methoxy bedeutet, gebildet.

Sofern $R_7'$ für Methoxy steht, kann diese Gruppe in an sich bekannter Weise durch Spaltung mit Jodwasserstoffsäure in die Hydroxygruppe überführt werden, welche anschließend in an sich bekannter Weise zu einer Sulfonylestergruppe Y verestert werden oder in eine Halogengruppe Y überführt werden kann. Ebenfalls können auf dieser Stufe gewünschtenfalls Nitrogruppen in an sich bekannter Weise in den Phenylteil des Ringgerüstes eingeführt werden.

In dem so erhaltenen Gemisch der isomeren Verbindungen der Formel IIa und IIIa

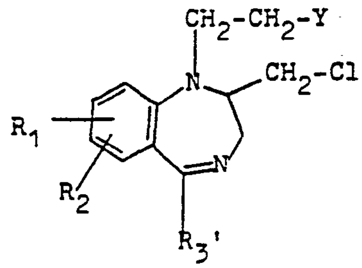

IIa

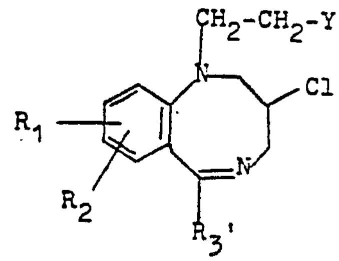

IIIa

worin $R_1$, $R_2$, $R_3'$ und Y obige Bedeutung besitzen, liegen die beiden isomeren Verbindungen in wechselnden Mengenverhältnissen vor in Abhängigkeit von der Art der Substituenten $R_1$, $R_2$ und $R_3'$. Dies ist jedoch für die folgende Umsetzung dieses Gemisches ohne Belang, da beide Isomere in einheitlicher Reaktion zu Verbindungen der Formel I cyclisiert werden können. Eine Trennung des isomeren Gemisches vor der weiteren Umsetzung ist daher nicht nötig. Selbstverständlich aber können gewünschtenfalls auch die Isomeren auf dieser Stufe getrennt und einzeln für die folgende Umsetzung eingesetzt werden.

Die Verbindungen der Formel IIa und IIIa lassen sich in an sich bekannter Weise in die entsprechenden N-Oxide überführen, beispielsweise unter den vorstehend für Verfahren b angegebenen Bedingungen.

Zur Herstellung von Verbindungen der Formel II, worin $R_3$ Pyrrolyl darstellt, werden Verbindungen der Formel IIa, worin $R_3'$ Furyl bedeutet, zunächst auf an sich bekannte Weise, beispielsweise in Analogie zu den in der DE-A-2 520 937 beschriebenen Verfahren, in die entsprechenden Dihydroxyverbindungen der Formel X

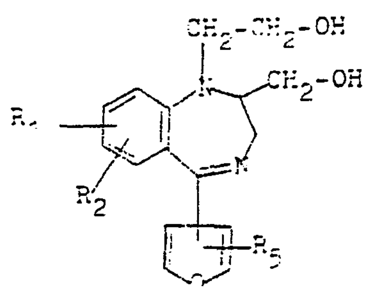

(X)

worin $R_1$, $R_2$ und $R_5$ obige Bedeutung besitzen, umgewandelt, und diese anschließend durch Umsetzung mit einem Amin $R_6$—$NH_2$ in die entsprechenden Pyrrolylverbindungen der Formel XI

(Siehe Formel Seite 8 f.)

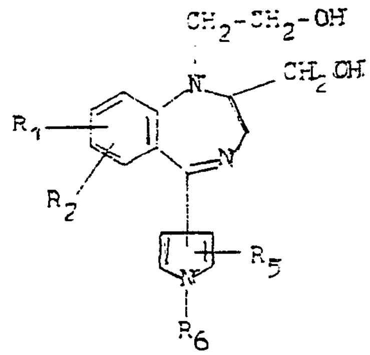

(XI)

worin $R_1$, $R_2$, $R_5$ und $R_6$ obige Bedeutung besitzen, überführt, beispielsweise unter den für Verfahren d angegebenen Reaktionsbedingungen. Die Hydroxygruppen in den Verbindungen der Formel XI können anschließend auf an sich bekannte Weise, beispielsweise durch Behandlung mit $POCl_3$ oder mit Triphenylphosphin/Tetrachlorkohlenstoff in Chlor überführt werden.

Zur Herstellung von Verbindungen der Formel II kann man auch von 2-Halogenbenzoyl-Verbindungen der Formel XII

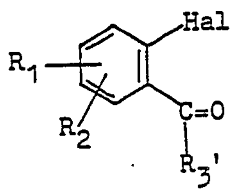

(XII)

worin $R_1$ und $R_2$ und $R_3'$ obige Bedeutung besitzen und Hal Halogen, vorzugsweise Chlor oder Fluor, bedeutet, ausgehen. Diese werden zunächst durch Umsetzung mit einer Propanoldiamin-Verbindung der Formel XIII

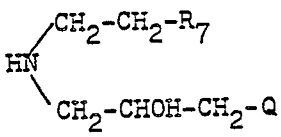

(XIII)

worin $R_7$ obige Bedeutung besitzt und Q für einen in eine Aminogruppe überführbaren Rest, beispielsweise eine geschützte Aminogruppe, steht, und anschließende Überführung der Gruppe Q in die freie Aminogruppe in Verbindungen der Formel XIV

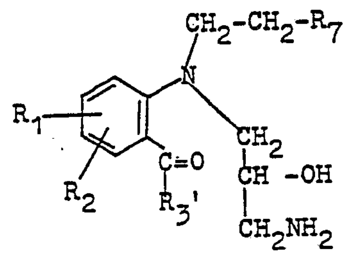

(XIV)

worin $R_1$, $R_2$ und $R_3'$ obige Bedeutung besitzen, überführt. Die Verbindungen der Formel XIV können auf an sich bekannte Weise durch Cyclisierung und anschließende Umwandlung der Gruppe $R_7$ in die Gruppe Y in Verbindungen der Formel II überführt werden. Dieses Verfahren eignet sich insbesondere zur Herstellung solcher Verbindungen der Formel II, worin der Phenylring des Benzodiazepingerüstes eine Nitrogruppe enthält.

Zur Herstellung von Verbindungen der Formel II kann auch von 2-Aminobenzoyl-Verbindungen der Formel XV

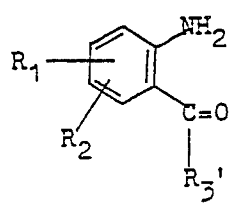

(XV)

worin $R_1$, $R_2$ und $R_3'$ obige Bedeutung besitzen, ausgegangen werden. In diesen Verbindungen wird zunächst auf an sich bekannte Weise die Aminogruppe durch eine $CH_2—CH_2—R_7$-Gruppe, worin $R_7$

obige Bedeutung besitzt, monosubstituiert und anschließend analog der in DE-A-2 810 349 beschriebenen Methode mit 1,2-Epoxypropylphthalimid umgesetzt. In den erhaltenen Verbindungen der Formel XVI

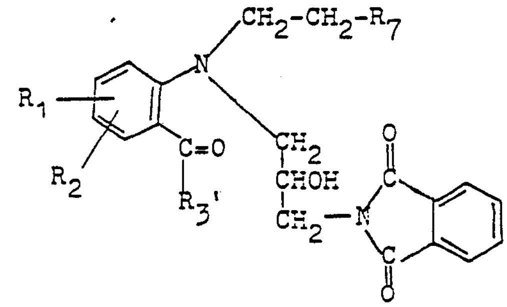

(XVI)

worin $R_1$, $R_2$, $R_3'$ und $R_7$ obige Bedeutung besitzen, wird anschließend in an sich bekannter Weise die Phthalimidgruppe gespalten, und die erhaltenen Aminoverbindungen werden in einem inerten Lösungsmittel, beispielsweise einem niederen Alkohol wie Methanol oder Essigsäure, zu Benzodiazocin-Verbindungen der Formel XVII

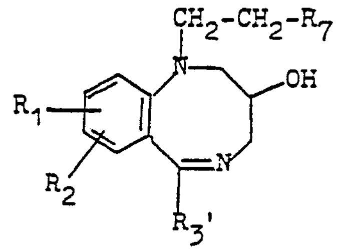

(XVII)

worin $R_1$, $R_2$, $R_3'$ und $R_7$ obige Bedeutung besitzen, cyclisiert. Die Verbindungen der Formel XVII können durch Behandlung mit $POCl_3$ in an sich bekannter Weise in die entsprechenden Benzodiazepinverbindungen der Formel II überführt werden.

Die erfindungsgemäßen heteroarylsubstituierten [1,2]-anellierten 1,4-Benzodiazepin-Derivate und ihre pharmakologisch akzeptablen Säureadditionssalze besitzen wertvolle pharmakologische Eigenschaften, und zeigen ein für 1,4-Benzodiazepine völlig neuartiges Wirkungsprofil.

So zeichnen sie sich von bisher bekannten phenylsubstituierten 1,4-Benzodiazepin-Derivaten, z. B. den aus der DE-A-2 835 708 bekannten ulcushemmenden Verbindungen oder als minor-Tranquilizer bekannten 5-Phenyl-1,4-benzodiazepin-2-onen wie Diazepam oder Chlordiazepoxid, dadurch aus, daß sie eine ausgeprägte neuroleptische Wirkungskomponente besitzen, während für Tranquilizer typische Wirkungen weitgehend zurückgedrängt sind.

So zeigen die erfindungsgemäßen Verbindungen starke Wirkungen in tierexperimentellen Standardtests, welche zum Nachweis neuroleptischer Wirkungen geeignet sind. Beispielsweise hemmen die Verbindungen in Mäusen in für Neuroleptica typischer Weise das durch Apomorphin induzierte (psychotische) Kletterverhalten. Zentralsedierende Eigenschaften sind dagegen in den Verbindungen nur in sehr viel geringerem Maße vorhanden, wie sich in Standardtests, z. B. durch Messung der Verlängerung der Hexobarbitalnarkosedauer in Mäusen zeigen läßt.

Die erfindungsgemäßen neuroleptisch wirksamen 1,4-Benzodiazepinderivate besitzen im neuroleptisch wirksamen Dosisbereich nur ein geringes Maß an sedierender Wirkung und zeichnen sich durch eine geringe Toxizität und große therapeutische Breite aus. Aufgrund ihres günstigen Wirkungsprofils sind sie für den Einsatz in der Behandlung von Erkrankungen des schizophrenen Formenkreises geeignet.

Das neuartige Wirkungsprofil der erfindungsgemäßen Verbindungen ist aus den Ergebnissen der nachstehend beschriebenen pharmakologischen Standardtests an Mäusen ersichtlich.

Beschreibung der pharmakologischen Untersuchungsmethoden.

1. Akute Toxizität.

Die akute 7-Tage-Toxizität wird nach einmaliger Applikation *per os* an der weißen nüchternen NMRI-Maus bestimmt und die $LD_{50}$-Werte über EDV durch eine Probit-Analyse errechnet.

2. Prüfung auf neuroleptische Eigenschaften.

Bestimmung der zur Hemmung des Apomorphin-induzierten Kletterverhaltens an der Maus wirksamen Dosis (modifizierte Methode nach P. Protais et al., Psychopharmacology *50,* (1976), 1-6).

Es werden Gruppen von jeweils 10 männlichen NMRI-Mäusen von 18-24 g Körpergewicht pro Testdosis verwendet. Die Testsubstanz wird *per os* in 2%iger Tyloselösung suspendiert appliziert.

Nach 60 Minuten werden den Tieren je 1,0 mg/kg Apomorphin s. c. injiziert und jedes Tier wird sofort nach der Injektion unter einen aufrecht stehenden Drahtgitterzylinder (Durchmesser 13 cm, Höhe 16 cm, oben verschlossen) gesetzt. Nach 10, 20 und 30 Minuten wird das Kletterverhalten der Tiere bewertet

nach folgendem Punktsystem :

0 Punkte = keine Pfote am Gitter

1 Punkt = ein oder zwei Pfoten berühren das Gitter

2 Punkte = drei oder alle Pfoten greifen das Gitter oder Klettern

Für jede Testgruppe wird die Summe der Punkte (Höchstpunktzahl = 20) zu den drei Testzeiten festgestellt und das Mittel aus den 3 Werten genommen, um die durch die Testsubstanz bewirkte Hemmung des Kletterverhaltens zu ermitteln.

In der nachfolgenden Tabelle ist die Hemmwirkung in % Hemmung im Vergleich zu dem Kletterverhalten einer nicht mit der Testsubstanz behandelten Kontrollgruppe angegeben.

3. Prüfung auf zentral-sedierende Eigenschaften

Bestimmung der zur Verlängerung der Hexobarbitalnarkose an der Maus wirksamen Dosis (modifizierte Methode nach J. W. Kemp et al., Arch. Int. Pharmacology *193*, (1971), 37-47).

Es werden Gruppen von jeweils 10 männlichen Mäusen von 22-26 g Körpergewicht pro Testdosis verwendet. Die Testsubstanz wird *per os* in 2 %iger Tyloselösung suspendiert appliziert.

Nach 60 Minuten werden den Tieren 50 mg/kg Hexobarbital-Natrium (= narkotische Dosis) i. v. injiziert. Es wird die Dauer des Verlustes des Aufrichtereflexes in zehntel Minuten gemessen. Die durch die Testsubstanz bewirkte Verlängerung dieser Dauer des Reflexverlustes wird durch einen Vergleich mit der Dauer des Reflexverlustes bei nur mit einer testsubstanzfreien Tyloselösung und Hexobarbital-Natrium behandelten Kontrolltieren ermittelt. In der nachstehenden Tabelle ist der durch die Testsubstanzen verursachte Verlängerungsfaktor angegeben.

Die folgende Tabelle gibt nach den vorstehend beschriebenen Testmethoden erhaltene Ergebnisse wieder. Die für die Verbindungen der Formel I angegebenen beispielsnummern beziehen sich auf die nachstehenden Herstellungsbeispiele.

(Siehe Tabelle Seite 11 f.)

Als Arzneimittel können die Verbindungen der Formel I und ihre physiologisch verträglichen Säureadditionssalze zusammen mit üblichen pharmazeutischen Hilfsstoffen in galenischen Zubereitungen verabreicht werden, wobei die Dosierung der zu behandelnden Spezies und den individuellen Erfordernissen angepaßt wird. Parenterale Formulierungen werden im allgemeinen weniger Aktivsubstanz enthalten als Präparate zu oraler Verabreichung. Als Beispiele galenischer Zubereitungsformen seien Tabletten, Kapseln, Pulver, Granulate, Dragees, Lösungen, Emulsionen, Suspensionen oder Suppositorien genannt. Diese galenischen Zubereitungen können nach an sich bekannten Methoden unter Verwendung gebräuchlicher pharmazeutischer Träger- und/oder Hilfsstoffe hergestellt werden. Feste Präparate können anorganische Trägerstoffe wie Talkum oder organische Trägerstoffe wie Milchzucker oder Stärke und gegebenenfalls weitere zur Tablettenherstellung übliche Hilfsstoffe wie zum Beispiel Gleitmittel, beispielsweise Magnesiumstearat, Bindemittel oder Tablettensprengmittel enthalten. Flüssige Präparate können die üblichen Verdünnungsmittel wie Wasser, flüssige fette Öle oder Paraffin sowie gegebenenfalls weitere Hilfsstoffe, z. B. Suspensionsmittel wie Polyoxyäthylenglykol, Emulgatoren und dergleichen enthalten.

Die nachfolgenden Beispiele erläutern die Herstellung der neuen Verbindungen der allgemeinen Formel I, sie sollen jedoch den Umfang der Erfindung in keiner Weise beschränken.

Die Strukturen den neuen Verbindungen werden durch spektroskopische Untersuchungen, insbesondere durch eine genaue Analyse der NMR-Spektren gesichert. Im IR-Spektrum wird die C=N-Bande im Bereich 1 600 bis 1 630 $cm^{-1}$ bestimmt. In der Tabelle werden bei Salzformen eventuell eingebundene Mengen an Wasser, Aceton, Äthanol oder dergleichen angeführt.

## Beispiel 1

1,2,4,4a-Tetrahydro-9-chlor-7-(3-thienyl)-5H-[1,4] oxazino-[4,3-a] [1,4] benzodiazepin.

A. Eine Lösung von 92 g $N_1$-(2-Methoxyäthyl)-$N_1$-(4-chlorphenyl)-2-hydroxy-1,3-diaminopropan und 40 g Triäthylamin in 600 ml Methylenchlorid wird bei Raumtemperatur tropfenweise mit einer Lösung von 52 g Thiophen-3-carbonylchlorid in 200 ml Methylenchlorid versetzt und das Reaktionsgemisch 15 Stunden lang reagieren lassen. Nach Aufarbeitung des Reaktionsgemisches wird das Reaktionsprodukt aus Isopropanol kristallisiert. Es werden 118 g $N_1$-(3-Thienylcarbonyl)-$N_2$-(2-methoxyäthyl)-$N_2$-(4-chlorphenyl)-2-hydroxy-1,3-diaminopropan erhalten. Schmelzpunkt 141 bis 142 °C.

B. 10 g der vorstehend beschriebenen Verbindung werden mit 10 ml Phosphoroxidtrichlorid versetzt und im Ölbad 16 Stunden bei 120 °C reagieren lassen. Das Reaktionsgemisch wird sodann mit Chloroform verdünnt, anschließend mit Eis und danach mit wässriger Natriumhydroxidlösung versetzt. Nach Aufarbeiten der organischen Phase wird das Rohprodukt aus Äther kristallisiert. Es werden 8 g 7-

| Testsubstanz der Formel I Beispiel Nr. | Hemmung des apomorphin-induzierten Kletterverhaltens | | Verlängerung der Hexobarbitalnarkosedauer | | Toxizität $LD_{50}$ mg/kg |
|---|---|---|---|---|---|
| | Dosis mg/kg | % Hemmung | Dosis mg/kg | Verlängerungsfaktor | |
| 22 | 8,1 | 50 | 37,7 | 1,5 | >811 |
| 35 | 9,8 | 35 | 9,8 | 2,1 | >311 |
| 10a | 13,3 | 31 | ≦ 42,2 | 0 | >422 |
| 36 | 22,3 | 34 | ≦ 70,4 | 0 | >704 |
| 63 | 21,3 | 11 | 67,2 | 1,5 | |
| 65 | 22,4 | 12 | | | |
| 9 | 4,2 | 48 | ≦ 41,5 | 0 | >893 |
| 41 | 13,9 | 14 | 43,8 | 1,6 | |
| 4 | 2,9 | 70 | ≦ 13,8 | 0 | ~ 440 |
| 51 | 2,2 | 57 | 22,3 | 1,9 | >704 |
| 55 | 0,2 | 47 | 6,8 | 1,3 | >315 |
| 69 | 0,7 | 71 | ≦ 28,0 | 0 | ~ 420 |
| 70 | 2,2 | 36 | ≦ 39,6 | 0 | ~ 790 |
| 74 | 3,3 | 70 | 33,0 | 2,0 | ~ 730 |

Chlor-1-(β-chloräthyl)-2-chlormethyl-5-(3-thienyl)-2,3-dihydro-1H-1,4-benzodiazepin erhalten. Schmelzpunkt 115 bis 116 °C.

C. Eine Lösung von 11 g 7-Chlor-1-(β-chloräthyl)-2-chlormethyl-5-(3-thienyl)-2,3-dihydro-1H-1,4-benzodiazepin in 60 ml Dioxan und 160 ml 4,5 %iger Natriumhydroxidlösung wird 5 Stunden unter Rückfluß erhitzt. Nach Abziehen des Lösungsmittels im Vakuum wird das Reaktionsprodukt aus Chloroform isoliert und anschließend an Aluminiumoxid der Aktivitätsstufe II mit Methylenchlorid chromatographiert. Nach Abziehen des Methylenchlorids werden 6,5 g 1,2,4,4a-Tetrahydro-9-chlor-7-(3-thienyl)-5H-[1,4] oxazino [4,3-a] [1,4] benzodiazepin als Öl erhalten. Dieses wird durch Umsetzen mit alkoholischer Chorwasserstofflösung in sein Hydrochlorid überführt, welches aus Isopropanol als Hydrochlorid · $0,3H_2O$ kristallisiert. Fp. : 207 bis 212 °C.

Beispiel 2

1,2,4,4a-Tetrahydro-10-methoxy-7-(2-furyl)-5H-[1,4] oxazino [4,3-a] [1,4] benzodiazepin.

A. 100 g $N_1$-(2-Hydroxyäthyl)-$N_1$-(3-methoxyphenyl)-2-hydroxy-1,3-diaminopropan werden in 800 ml Chloroform in Gegenwart von 45,8 g Triäthylamin mit 54,4 g Furan-2-carbonylchlorid umgesetzt. Nach Aufarbeiten des Reaktionsgemisches wird das Reaktionsprodukt aus Isopropanol kristallisiert. Es werden 120 g $N_1$-(2-Furylcarbonyl)-$N_2$-(2-hydroxyäthyl)-$N_2$-(3-methoxyphenyl)-2-hydroxy-1,3-diaminopropan erhalten. Fp. : 92 bis 94 °C.

B. 30 g der vorstehenden Amidverbindung werden in 40 ml Phosphoroxidtrichlorid 2 Stunden im Ölbad bei 135 °C Badtemperatur reagieren lassen. Anschließend wird mit Chloroform verdünnt, und die Lössung nacheinander mit Eis und mit Natriumhydroxidlösung behandelt. Die organische Phase wird abgetrennt und aufgearbeitet. Es werden 25,4 g eines öligen Gemisches erhalten, welches ca. 20 % 9-Methoxy-1-(β-chloräthyl)-3-chlor-6-(2-furyl)-1,2,3,4-tetrahydro-1,5-benzodiazocin und ca. 80 % 8-Methoxy-(β-chloräthyl)-2-chlormethyl-5-(2-furyl)-2,3-dihydro-1H-1,4-benzodiazepin enthält. Dieses wird ohne weitere Trennung für die folgende Umsetzung eingesetzt. Gewünschtenfalls kann durch Kristallisation aus Äther die Benzodiazepinkomponente mit Schmelzpunkt 105 bis 106 °C kristallin abgetrennt werden.

C. Eine Lösung von 25 g des vorgehend beschriebenen isomeren Gemisches in 125 ml Dioxan und 325 ml 4,5 %iger Natriumhydroxidlösung wird 2 Stunden unter Rückfluß erhitzt. Nach Abziehen des Lösungsmittels im Vakuum wird das Reaktionsprodukt aus Chloroform isoliert und anschließend an Aluminiumoxid der Aktivitätsstufe II mit Methylenchlorid chromatographiert. Nach Abziehen des Methylenchlorids wird das Produkt aus Äther kristallisiert. Es werden 6,7 g 1,2,4,4a-Tetrahydro-10-methoxy-7-(2-furyl)-5H-[1,4] oxazino [4,3-a] [1,4] benzodiazepin erhalten. Fp. : 130 bis 132 °C.

Beispiel 3

1,2,4,4a-Tetrahydro-10-methoxy-7-(2-furyl)-5H-[1,4] oxazino-[4,3a] [1,4] benzodiazepin-6-oxid.

Eine Lösung von 6 g 1,2,4,4a-Tetrahydro-10-methoxy-7-(2-furyl)-5H-[1,4] oxazino [4,3-a] [1,4] benzodiazepin (Herstellung siehe Beispiel 2) in 100 ml Methylenchlorid wird mit 4,2 g 3-Chlorperbenzoesäure 3 Stunden unter Rückfluß erhitzt. Anschließend wird die Reaktionslösung mit wässriger verdünnter Natriumhydroxidlösung alkalisch gestellt und in üblicher Weise aufgearbeitet. Das isolierte Rohprodukt wird durch Chromatographie an Aluminiumoxid der Aktivitätsstufe II mit Methylenchlorid gereinigt und aus Isopropanol kristallisiert. Es werden 2,3 g 1,2,4,4a-Tetrahydro-10-methoxy-7-(2-furyl)-5H-[1,4] oxazino [4,3-a] [1,4]-benzodiazepin-6-oxid erhalten, Fp. : 183 bis 186 °C.

Beispiel 4

1,2,3,4,4a,5-Hexahydro-3-methyl-7-(2-thienyl)-pyrazino-[1,2-a] [1,4] benzodiazepin.

A. 170 g $N_1$-(2-Hydroxyäthyl)-$N_1$-phenyl-2-hydroxy-1,3-diaminopropan werden in Gegenwart von 89 g Triäthylamin in 800 ml Chloroform mit 118,7 g Thiophen-2-carbonylchlorid umgesetzt. Nach Aufarbeitung des Reaktionsgemisches wird das Reaktionsprodukt aus Isopropanol kristallisiert. Es werden 190 g $N_1$-(2-Thienylcarbonyl)-$N_2$-(2-hydroxyäthyl)-$N_2$-phenyl-2-hydroxy-1,3-diaminopropan erhalten, Fp. : 141 bis 142 °C.

B. 60 g der vorstehenden Amid-Verbindung werden in 70 ml Phosphoroxidtrichlorid 2 Stunden im Ölbad bei 140 °C Ölbadtemperatur reagieren lassen. Anschließend wird das Reaktionsgemisch nacheinander mit Eis und mit Natriumhydroxidlösung behandelt und wie üblich aufgearbeitet und das Reaktionsprodukt aus Chloroform isoliert. Es werden 51,0 g eines öligen Gemisches aus ca. 90 % 1-(β-Chloräthyl)-2-chlormethyl-5-(2-thienyl)-2,3-dihydro-1H-1,4-benzodiazepin und ca. 10 % 1-(β-Chloräthyl)-3-

chlor-6-(2-thienyl)-1,2,3,4-tetrahydro-1,5-benzodiazocin erhalten, welches ohne weitere Reinigung weiter umgesetzt wird.

C. 20 g des vorstehend beschriebenen Gemisches werden in 300 ml Methanol mit 20 g Methylamin bei 95 °C im Autoklaven 14 Stunden reagieren lassen. Anschließend wird das Reaktionsgemisch wie üblich aufgearbeitet und das Reaktionsprodukt durch Chromatographie an Aluminiumoxid der Aktivitätsstufe II mit Methylenchlorid/Chloroform gereinigt und dann aus Äther kristallisiert. Man erhält 12,8 g 1,2,3,4,4a,5-Hexahydro-3-methyl-7-(2-thienyl)-pyrazinol [1,2a] [1,4] benzodiazepin, Fp. : 124 bis 125 °C.

Beispiel 5

1,2,3,4,4a,5-Hexahydro-9-chlor-3-methyl-7-(2-furyl)-pyrazino [1,2-a] [1,4] benzodiazepin-6-oxid.

A. 118,3 g $N_1$-(2-Methoxyäthyl)-$N_1$-(4-chlorphenyl)-2-hydroxy-1,3-diaminopropan werden in 1 000 ml Chloroform in Gegenwart von 51,0 g Triäthylamin mit 61,0 g Furan-2-carbonylchlorid umgesetzt. Nach Aufarbeitung der Reaktionslösung wird das Reaktionsprodukt aus Isopropanol kristallisiert. Es werden 147 g $N_1$-(2-Furylcarbonyl)-$N_2$-(2-methoxyäthyl)-$N_2$-(4-chlorphenyl)-2-hydroxy-1,3-diaminopropan erhalten, Fp. : 121 bis 123 °C.

B. 146 g der vorstehenden Amidverbindung werden in 150 ml Phosphoroxidtrichlorid im Ölbad bei einer Ölbadtemperatur von 120 °C 4 Stunden reagieren lassen, anschließend wird das Reaktionsgemisch nacheinander mit Eis und mit wässriger Natriumhydroxidlösung behandelt und aufgearbeitet. Das Reaktionsprodukt wird aus Chloroform isoliert. Es werden 106,5 g eines öligen Gemisches von 7-Chlor-1-(β-chloräthyl)-2-chlormethyl-5-(2-furyl)-2,3-dihydro-1H-1,4-benzodiazepin und 3,8-Dichlor-1-(β-chloräthyl)-6-(2-furyl)-1,2,3,4-tetrahydro-1,5-benzodiazocin erhalten.

Bei Behandeln des vorstehenden Gemisches mit Äther kristallisieren 47,8 g der vorstehenden Benzodiazepinverbindung mit einem Schmelzpunkt von 90 bis 92 °C aus.

Aus der Mutterlauge erhält man nach Abziehen des Lösungsmittels im Vakuum und Reinigung des verbleibenden Rückstandes an Aluminiumoxid der Aktivitätsstufe II bis III mit Methylenchlorid 43 g eines Gemisches aus etwa gleichen Teilen des Benzodiazepin- und des Benzodiazocinisomeren. Dieses Gemisch wird in 215 ml Tetrachloräthan 1 Stunde unter Rückfluß erhitzt. Nach Abziehen des Lösungsmittels im Vakuum wird das Reaktionsprodukt mit wässriger Natriumhydroxidlösung behandelt und erneut chromatographisch an Aluminiumoxid gereinigt und anschließend aus Äther kristallisiert. Es werden weitere 34 g der Benzodiazepin-Verbindung mit Schmelzpunkt 92 bis 94 °C erhalten.

C. Eine Lösung von 17,8 g 7-Chlor-1-(β-chloräthyl)-2-chlormethyl-5-(2-furyl)-2,3-dihydro-1H-1,4-benzodiazepin in 250 ml Methylenchlorid werden mit 10,4 g 3-Chlorperbenzoesäure 2 Stunden unter Rückfluß erhitzt. Anschließend wird die Reaktionslösung mit wässriger verdünnter Natriumhydroxidlösung alkalisch gemacht und in üblicher Weise aufgearbeitet, das Reaktionsprodukt isoliert, anschließend durch Chromatographie an Aluminiumoxid der Aktivitätsstufe II mit Methylenchlorid gereinigt und aus Methanol kristallisiert. Es werden 13 g 7-Chlor-1-(β-chloräthyl)-2-chlormethyl-5-(2-furyl)-2,3-dihydro-1H-1,4-benzodiazepin-4-oxid erhalten, Fp. : 158 bis 159 °C.

D. 12 g der vorstehenden N-Oxid-Verbindung werden in 150 ml Methanol mit 12 g Methylamin bei 95 °C im Autoklaven während 14 Stunden reagieren lassen. Nach Aufarbeiten des Reaktionsgemisches wird das erhaltene Rohprodukt durch Chromatographie an Aluminiumoxid der Aktivitätsstufe II mit Methylenchlorid/Chloroform gereinigt und aus Isopropanol kristallisiert. Man erhält 7,3 g 1,2,3,4,4a,5-Hexahydro-9-chlor-3-methyl-7-(2-furyl)-pyrazino [1,2-a] [1,4] benzodiazepin-6-oxid, Fp. : 202 bis 204 °C.

Beispiel 6

1,2,4,4a-Tetrahydro-9-chlor-7-(2-furyl)-5H-[1,4] thiazino-[4,3-a] [1,4] benzodiazepin.

Eine Lösung von 12,7 g 7-Chlor-1-(β-chloräthyl)-2-chlormethyl-5-(2-furyl)-2,3-dihydro-1H-1,4-benzodiazepin (oder einer Mischung aus diesem und dem dazu isomeren 3,8-Dichlor-1-(β-chloräthyl)-6-(2-furyl)-1,2,3,4-tetrahydro-1,5-benzodiazocin, Herstellung siehe Beispiel 5B) in 150 ml Dioxan wird mit 8,5 g Dinatriumsulfid-nonahydrat in 75 ml Wasser versetzt und das Reaktionsgemisch 5 Stunden unter Rückfluß erhitzt. Nach Abziehen des Lösungsmittels im Vakuum wird das rohe Reaktionsprodukt aus Chloroform isoliert und anschließend durch Chromatographie an Aluminiumoxid der Aktivitätsstufe II mit Methylenchlorid gereinigt und aus Äther kristallisiert. Es werden 9,7 g 1,2,4,4a-Tetrahydro-9-chlor-7-(2-furyl)-5H-[1,4]-thiazino [4,3-a] [1,4] benzodiazepin erhalten, Fp. : 138 bis 140 °C.

Beispiel 7

1,2,3,4,4a,5-Hexahydro-9-chlor-3-methyl-7-(2-furyl)-pyrazino [1,2-a] [1,4] benzodiazepin.

3,4 g 7-Chlor-1-(β-chloräthyl)-2-chlormethyl-5-(2-furyl)-2,3-dihydro-1H-1,4-benzodiazepin werden in 100 ml Methanol mit 0,8 g Natriumhydroxid in 1 ml Wasser und 2,5 g Methylamin bei 95 °C im Autoklaven 5 Stunden reagieren lassen. Anschließend wird das Reaktionsgemisch wie üblich aufgearbeitet und das Reaktionsprodukt durch Chromatographie an Aluminiumoxid der Aktivitätsstufe II mit Methylenchlorid/Chloroform gereinigt, wobei die das mittelpolare Produkt enthaltenden Fraktionen abgetrennt werden und das daraus erhaltene 1,2,3,4,4a,5-Hexahydro-9-chlor-3-methyl-7-(2-furyl)-pyrazino [1,2-a] [1,4] benzodiazepin in Isopropanol gelöst und mit alkoholischer Chlorwasserstoffsäure in sein Salz überführt wird. Dieses kristallisiert aus Isopropanol als Dihydrochloridhemihydrat mit 0,1 mol Isopropanol, Fp. : > 240 °C, Ausbeute 0,8 g.

Beispiel 8

1,2,3,4,4a,5-Hexahydro-9-chlor-3-methyl-7-(2-furyl)-pyrazino [1,2-a] [1,4] benzodiazepin.

1 g 7-Chlor-1-(β-chloräthyl)-2-chlormethyl-5-(2-furyl)-2,3-dihydro-1H-1,4-benzodiazepin werden in 40 ml Methanol mit 0,8 Methylamin bei 50 °C 3 Stunden reagieren lassen. Anschließend wird das Reaktionsgemisch wie in Beispiel 7 beschrieben aufgearbeitet. Bei der chromatographischen Reinigung werden die die stärker polare Titelverbindung enthaltenden Fraktionen abgetrennt. Man erhält daraus 0,4 g 1,2,3,4,4a,5-Hexahydro-9-chlor-3-methyl-7-(2-furyl)-pyrazino [1,2-a] [1,4] benzodiazepin.

Beispiel 9

1,2,3,4,4a,5-Hexahydro-9-chlor-3-methyl-7-[2-(N-methyl)-pyrrolyl]-pyrazino [1,2-a] [1,4] benzodiazepin.

19 g 7-Chlor-1-(β-chloräthyl)-2-chlormethyl-5-(2-furyl)-2,3-dihydro-1H-1,4-benzodiazepin werden in 300 ml Methanol mit 15 g Methylamin bei 95 °C im Autoklaven während 14 Stunden reagieren lassen. Nach Aufarbeitung des Reaktionsgemisches und chromatographischer Reinigung des Rohproduktes an Aluminiumoxid der Aktivitätsstufe II mit Methylenchlorid/Chloroform wird das erhaltene 1,2,3,4,4a,5-Hexahydro-9-chlor-3-methyl-7-[2-(N-methyl) pyrrolyl]-pyrazino [1,2-a] [1,4] benzodiazepin in Äthanol mit äthanolischer Chlorwasserstoffsäure in sein Salz überführt. Dieses kristallisiert aus Äthanol/Äther als Dihydrochlorid mit 0,75 mol Wasser, Fp. : > 230 °C, Ausbeute : 13,4 g.

Beispiel 10

a) 1,2,3,4,4a,5-Hexahydro-3,9-dimethyl-7-(2-furyl)-pyrazino [1,2-a] [1,4] benzodiazepin und

b) 1,2,3,4,4a,5-Hexahydro-3,9-dimethyl-7-(5-chlor-2-furyl)-pyrazino [1,2-a] [1,4] benzodiazepin.

9,3 g 1,2,3,4,4a,5-Hexahydro-3,9-dimethyl-7-(2-furyl)-pyrazino [1,2-a] [1,4] benzodiazepin-6-oxid (hergestellt analog Beispiel 5) werden in 150 ml Chloroform mit 17 ml Phosphortrichlorid 1 Stunde unter Rückfluß erhitzt. Anschließend wird das Reaktionsgemisch mit wässriger Natriumhydroxidlösung behandelt und aufgearbeitet. Das erhaltene Rohprodukt wird durch Chromatographie an Aluminiumoxid der Aktivitätsstufe II mit Methylenchlorid/Chloroform getrennt. Hierbei fallen zunächst in den Vorfraktionen 1,7 g des weniger polaren 1,2,3,4,4a,5-Hexahydro-3,9-dimethyl-7-(5-chlor-2-furyl)-pyrazino [1,2-a] [1,4] benzodiazepin an, welches als Öl (IR : 1 615 $cm^1$ C=N) isoliert wird (b). Danach werden 5 g des stärker polaren 1,2,3,4,4a,5-Hexahydro-3,9-dimethyl-7-(2-furyl)-pyrazino [1,2-a] [1,4] benzodiazepin (a) erhalten.

Zur Überführung in sein Salz wird das 1,2,3,4,4a,5-Hexahydro-3,9-dimethyl-7-(2-furyl)-pyrazino [1,2-a] [1,4] benzodiazepin in Isopropanol mit Chlorwasserstoffgas behandelt. Aus Isopropanol/Äther kristallisiert das Dihydrochlorid-trihydrat, Fp. : 210 °C (Zersetzung).

Beispiel 11

1,2,3,4,4a,5-Hexahydro-9-nitro-3-methyl-7-(2-thienyl)-pyrazino [1,2-a] [1,4] benzodiazepin.

A. Eine Lösung von 15,5 g 1-(β-Chloräthyl)-2-chlormethyl-5-(2-thienyl)-2,3-dihydro-1H-1,4-benzodiazepin in 110 ml Acetanhydrid wird bei 45 °C portionsweise mit 12 g Kupfer-II-nitrat-trihydrat versetzt. Nach beendeter Reaktion wird das Reaktionsgemisch mit Eiswasser versetzt und alkalisch aufgearbeitet. Das Reaktionsprodukt wird am Aluminiumoxid der Aktivitätsstufe II mit Methylenchlorid chromatographisch gereinigt. Man erhält 7,3 g 7-Nitro-1-(β-chloräthyl)-2-chlormethyl-5-(2-thienyl)-2,3-dihydro-1H-1,4-benzodiazepin als Öl.

B. 2,7 g der vorstehenden Verbindung werden in 100 ml Methanol mit 10 g Methylamin bei 95 °C im Autoklaven 14 Stunden reagieren lassen. Nach Aufarbeitung des Reaktionsgemisches wird das Reaktionsprodukt chromatographisch an Aluminiumoxid der Aktivitätsstufe II mit Methylenchlorid/Chloroform gereinigt und aus Äther kristallisiert. Man erhält 1,1 g 1,2,3,4,4a,5-Hexahydro-9-nitro-3-methyl-7-(2-thienyl)-pyrazino [1,2-a] [1,4] benzodiazepin, Fp. : 174 bis 175 °C.

Beispiel 12

1,2,3,4,4a,5-Hexahydro-3-n-butyl-9-methyl-7-(2-furyl)-pyrazino [1,2-a] [1,4] benzodiazepin-6-oxid.

4 g 7-Methyl-1-(β-chloräthyl)-2-chlormethyl-5-(2-furyl)-2,3-dihydro-1H-1,4-benzodiazepin-4-oxid (hergestellt analog Beispiel 5A-C) werden in 10 ml n-Butylamin gelöst, und die Lösung 14 Stunden im Autoklaven auf 90 °C erhitzt. Nach Aufarbeiten des Reaktionsgemisches wird das erhaltene Rohprodukt durch Chromatographie an Aluminiumoxid der Aktivitätsstufe II mit Methylenchlorid/Chloroform gereinigt und aus Äther kristallisiert. Man erhält 2,1 g 1,2,3,4,4a,5-Hexahydro-3-(n-butyl)-9-methyl-7-(2-furyl)-pyrazino [1,2-a] [1,4] benzodiazepin-6-oxid, Fp. : 104 bis 106 °C.

Beispiel 13

1,2,4,4a-Tetrahydro-9-chlor-7-[2-(N-methyl)-pyrrolyl]-5H-[1,4] oxazino [4,3-a] [1,4] benzodiazepin.

2 g 1,2,4,4a-Tetrahydro-9-chlor-7-(2-furyl)-5H-[1,4]-oxazino [4,3-a] [1,4] benzodiazepin (hergestellt analog Beispiel 2) werden mit 1,8 g Methylamin-hydrochlorid und 4 g Methylamin in 100 ml Methanol 4 Stunden im Autoklaven auf 95 °C erhitzt. Nach Abziehen des Lösungsmittels wird das Rohprodukt aus Chloroform isoliert, anschließend durch Chromatographie an Aluminiumoxid der Aktivitätsstufe II mit Methylenchlorid gereinigt und dann aus Hexan kristallisiert. Man erhält 1,5 g 1,2,4,4a-Tetrahydro-9-chlor-7-[2-(N-methyl) pyrrolyl]-5H-[1,4] oxazino [4,3-a] [1,4]-benzodiazepin, Fp. : 95 bis 97 °C.

Beispiel 14

1,2,4,4a-Tetrahydro-9,10-äthylendioxy-7-(2-thienyl)-5H-[1,4] oxazino-[4,3-a] [1,4] benzodiazepin.

A. 29,6 g $N_1$-(2-thienylcarbonyl)-$N_2$-(2-hydroxyäthyl)-$N_2$-(3,4-äthylendioxyphenyl)-2-hydroxy-1,3-diaminopropan werden in 30 ml Phosphoroxidtrichlorid 14 Stunden im Ölbad bei 120 °C bar-Temperatur reagieren gelassen. Anschließend wird mit Chloroform verdünnt, und die Lösung nacheinander mit Eis und mit Natriumhydroxidlösung behandelt. Die organische Phase wird abgetrennt und aufgearbeitet. Das erhaltene Reaktionsprodukt verfestigt sich nach Abziehen des Lösungsmittels. Durch Kristallisation aus Isopropanol erhält man 25,6 g 7,8-Äthylendioxy-1-(beta-chloräthyl)-2-chlormethyl-5-(2-thienyl)-2,3-dihydro-1H-1,4-benzodiazepin, Fp. : 184-187 °C.

B. Eine Lösung von 13 g des vorgehend beschriebenen Produktes in 60 ml Dioxan wird mit 35 ml 20 %iger Natriumhydroxid-Lösung und 100 ml Wasser 4 Stunden unter Rückfluß erhitzt. Nach Abziehen des Lösungsmittels im Vakuum wird das Reaktionsprodukt aus Chloroform isoliert und anschließend an Aluminiumoxid der Aktivitätsstufe II mit Methylenchlorid chromatographiert. Nach Abziehen des Methylenchlorids wird das Produkt aus Äther kristallisiert. Es werden 5,5 g 1,2,4,4a-Tetrahydro-9,10-äthylendioxy-7-(2-thienyl)-5H-[1,4] oxazino [4,3-a] [1,4] benzodiazepin erhalten. Fp. : 192-194 °C.

Nach den in den Beispielen 1-14 beschriebenen Verfahren können auch die in der folgenden Tabelle aufgeführten [1,2]-annellierten 7-Heteroaryl-1,4-benzodiazepin-Verbindungen der Formel I, ausgehend von entsprechenden Verbindungen der Formel II oder III, hergestellt werden.

(Siehe Tabellen Seite 16 ff.)

Beispiel I : Tabletten

Man stellt Tabletten in folgender Zusammensetzung pro Tablette her :

| | |
|---|---|
| 1,2,3,4,4a,5-Hexahydro-3-methyl-7-(2-thienyl)-pyrazino [1,2-a] [1,4] benzodiazepin | 25 mg |
| Maisstärke | 60 mg |
| Milchzucker | 130 mg |
| Gelatine (10 %ige Lösung) | 6 mg |

Der Wirkstoff, die Maisstärke und der Milchzucker werden mit der 10 %igen Gelatinelösung eingedickt. Die Paste wird zerkleinert und das entstandene Granulat wird auf ein geeignetes Blech

| Bsp. Nr. | $R_1$ | $R_2$ | $R_3$ | X | n | Salz | Fp °C |
|---|---|---|---|---|---|---|---|
| 15 | H | H | 2-thien. | O | 0 | Base | 160-162 |
| 16 | 9-Cl | H | 2-fur. | O | 0 | Base | 143-145 |
| 17 | 9-$C_2H_5O$ | H | 3-fur. | O | 0 | HCl | 224-226 |
| 18 | 9-$CH_3$ | H | 2-thien. | O | 0 | HCl · 0,5$H_2O$ | 167-171 |
| 19 | 9-$CH_3$ | H | 2-fur. | O | 0 | Base | 129-131 |
| 20 | 9-$CH_3$ | H | 3-fur. | O | 1 | Base | 190-192 |
| 21 | 9-$CH_3$ | H | 3-fur. | O | 0 | Base | 121-122 |
| 22 | 9-$CH_3$ | H | 5-Br-2-thien. | O | 0 | Base | 117-120 |
| 23 | 10-$CH_3O$ | H | 2-fur. | S | 1 | Base | 154-156 |
| 24 | H | H | 2-thien. | S | 1 | Base | 188-191 |
| 25 | 9-$CH_3$ | H | 2-thien. | S | 0 | Base | 100-103 |
| 26 | 9-$CH_3$ | H | 2-fur. | S | 0 | Base | 128-130 |

thien = Thienyl, fur. = Furyl, pyrrol. = Pyrrolyl
HCl = Hydrochlorid, Base = freie Base, p-Tos. = p-Toluolsulfonat

| Bsp. Nr. | $R_1$ | $R_2$ | $R_3$ | X | n | Salz | Fp °C |
|---|---|---|---|---|---|---|---|
| 27 | 9-$CH_3$ | H | 3-fur. | S | 0 | Base | 105-107 |
| 28 | 9-$CH_3$ | H | 5-Br-2-thien. | S | 0 | HCl | 205 (Z) |
| 29 | H | H | 2-thien. | N-$CH_3$ | 1 | Base | 208-211 |
| 30 | 9-Cl | H | 2-thien. | N-$CH_3$ | 0 | Base | 115-117 |
| 31 | 9-Cl | H | 2-thien. | N-$CH_3$ | 1 | Base | 199-201 |
| 32 | 9-Cl | H | 3-thien. | N-$CH_3$ | 0 | 2HCl · 0,5$H_2$O | > 240 |
| 33 | H | H | 2-thien. | N-H | 1 | Base | 204-206 |
| 34 | 9-$CH_3$ | H | 2-thien. | N-$CH_3$ | 0 | Base | 117-118 |
| 35 | 9-$CH_3$ | H | 2-fur. | N-$CH_3$ | 1 | Base | 182-184 |
| 36 | 10-$CH_3$O | H | 2-fur. | N-$CH_3$ | 1 | Base | 189-191 |
| 37 | 9-$CH_3$ | H | N-$CH_3$-2-pyrrol. | N-$CH_3$ | 0 | Base | 93- 95 |
| 38 | 9-$CH_3$ | H | N-(-n-$C_4H_9$)-2-pyrrol. | N-n-$C_4H_9$ | 0 | Base | Öl |

öl = ölig,  Z = Zersetzung,  S = sintern,

| Bsp. Nr. | $R_1$ | $R_2$ | $R_3$ | X | n | Salz | Fp °C |
|---|---|---|---|---|---|---|---|
| 39 | 9-$CH_3$ | H | 5-Br-2-thien. | N-$CH_3$ | 0 | 2HCl·0,5$H_2$O ·1$(CH_3)_2$CHOH | 200 (Z) |
| 40 | 9-$NO_2$ | H | 2-thien. | N-$CH_3$ | 1 | Base | Öl |
| 41 | 9-$C_2H_5$O | H | N-$CH_3$-3-pyrrol. | N-$CH_3$ | 0 | 2HCl·1,5$H_2$O | >240 |
| 42 | 9-$NO_2$ | H | 2-thien. | O | 0 | Base | Öl |
| 43 | 11-$NO_2$ | H | 2-thien. | O | 0 | Base | 125-128 |
| 44 | H | H | 3-Cl-2-thien. | N-$CH_3$ | 0 | Base | 121-123 |
| 45 | H | H | 3-$CH_3$-2-thien. | N-$CH_3$ | 0 | 2 p-Tos. | 193-196 |
| 46 | 9-F | H | 2-thien. | N-$CH_3$ | 0 | 2HCl·1,5$H_2$O | >240 |
| 47 | 10-$CH_3$O | H | 2-fur. | N-$CH_3$ | 0 | Base | 121-122 |
| 48 | 10-$CH_3$O | H | 2-fur. | NH | 1 | Base | 182-184 |
| 49 | 9-$C_2H_5$O | H | 2-thien. | N-$CH_3$ | 0 | 2HCl | >240 |
| 50 | 9-$CH_3$S | H | 2-thien. | N-$CH_3$ | 0 | Base | 149-151 |

| Bsp. Nr. | $R_1$ | $R_2$ | $R_3$ | X | n | Salz | Fp °C |
|---|---|---|---|---|---|---|---|
| 51 | 10-$CH_3O$ | H | 2-thien. | N-$CH_3$ | 0 | Base | 126-128 |
| 52 | 9-$(CH_3)_2CH$ | H | 2-thien. | N-$CH_3$ | 0 | Base | 169-170 |
| 53 | H | H | 2-thien. | NH | 0 | Base | 123-126 |
| 54 | 9,10-O-$CH_2$-O | | 2-thien. | N-$CH_3$ | 0 | Base·0,25$H_2O$ | 217-219 |
| 55 | 10-F | H | 2-thien. | N-$CH_3$ | 0 | Base | 111-113 |
| 56 | H | H | 2-thien. | S | 0 | Base | 149-150 |
| 57 | H | H | N-$CH_3$-2-pyrrol. | N-$CH_3$ | 0 | Base | 149-152 |
| 58 | H | H | 5-$CH_3$-2-fur. | N-$CH_3$ | 1 | Base | 153-155 |
| 59 | 9,10-di-$CH_3$ | | 2-thien. | N-$CH_3$ | 0 | 2HCl·1,5$H_2O$ | 230 (Z) |
| 60 | H | H | 5-Cl-2-fur. | N-$CH_3$ | 1 | HCl | 230 (Z) |
| 61 | H | H | 4-Br-2-thien. | N-$CH_3$ | 0 | Base | 139-140 |
| 62 | 10-$CH_3$ | H | 2-thien. | N-$CH_3$ | 0 | 2HCl·2$H_2O$ ·0,2$(CH_3)_2CHOH$ | 218 (Z) |

| Bsp. Nr. | $R_1$ | $R_2$ | $R_3$ | X | n | Salz | Fp °C |
|---|---|---|---|---|---|---|---|
| 63 | 10,8-di-$CH_3$ | | 2-thien. | O | 0 | Base | 144-147 |
| 64 | 10,8-di-$CH_3$ | | 2-thien. | N-$CH_3$ | 0 | 2HCl·1$H_2O$ | >240 |
| 65 | 10,8-di-$CH_3$ | | 2-thien. | S | 0 | Base | 130-132 |
| 66 | 9-$CF_3$ | H | 2-thien. | N-$CH_3$ | 0 | | |
| 67 | H | H | 3-fur. | O | 0 | Base | 159-161 |
| 68 | 10,8-di-$CH_3O$ | | 2-thien. | N-$CH_3$ | 0 | Base | 151-152 |
| 69 | H | H | 2-thien. | N-$C_2H_5$ | 0 | 2HCl·1,5$H_2O$ | >240 |
| 70 | H | H | 2-thien. | N-$CH_2$-CH=$CH_2$ | 0 | 2HCl | >240 |
| 71 | H | H | 2-thien. | N-CH-$(CH_3)_2$ | 0 | 2HCl·1H2O | 195 (S) |
| 72 | H | H | 2-thien. | N-C$(CH_3)_3$ | 0 | Base | 142-143 |
| 73 | H | H | 2-thien. | N-(n-$C_4H_9$) | 0 | Base | 140-141 |
| 74 | H | H | 2-thien. | N-$(CH_2)_2$CH$(CH_3)_2$ | 0 | 2HCl·$H_2O$ ·0,66$(CH_3)_2$CHOH | 180 (S) |

| Bsp. Nr. | $R_1$ | $R_2$ | $R_3$ | X | n | Salz | Fp °C |
|---|---|---|---|---|---|---|---|
| 75 | H | H | 2-thien. | $N-CH_2-CH_2OCH_3$ | 0 | Base | 71- 73 |
| 76 | H | H | 2-thien. | $N-(CH_2)_3OCH_3$ | 0 | Base | 97- 98 |
| 77 | H | H | 2-thien. | $N-CH_2-CH_2OH$ | 0 | $2HCl \cdot 1,5H_2O$ | 185 (S) |
| 78 | H | H | 2-thien. | $N-(CH_2)_3-OH$ | 0 | Base | 139-141 |
| 79 | H | H | $N-[(CH_3)_3C-CH_2]-$ 2-pyrrol. | 0 | 0 | Base | Öl |
| 80 | H | H | $N-(CH_3OCH_2-CH_2-CH_2)$ 2-pyrrol. | 0 | 0 | Base | 195-199 |
| 81 | H | H | 2-thien. | $N-CH_2-$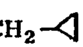 | 0 | Base | 129-131 |
| 82 | 8,10-di-Cl | | 2-thien. | $N-CH_3$ | 0 | | |
| 83 | $9-(CH_3)_3C$ | H | 2-thien. | $N-CH_3$ | 0 | Base | 204-206 |
| 84 | $9-(CH_3)_3C$ | H | 2-thien. | $N-CH_3$ | 0 | 2HCl | 240 |
| 85 | $9,10-OCH_2-CH_2-O$ | | 2-thien. | $N-CH_3$ | 0 | Base | 225-228 |
| 86 | $10-C_2H_5$ | H | 2-thien. | $N-CH_3$ | 0 | Base | Öl |
| | | | | | | | 114-116 |

gebracht und bei 45 °C getrocknet. Das getrocknete Granulat wird durch eine Zerkleinerungsmaschine geleitet und in einem Mixer mit weiteren folgenden Hilfsstoffen vermischt :

| | |
|---|---|
| Talkum | 5 mg |
| Magnesiumstearat | 5 mg |
| Maisstärke | 9 mg |

und sodann zu Tabletten von 240 mg verpreßt.

**Patentansprüche** (für die Vertragsstaaten : BE, CH, DE, FR, GB, IT, LI, LU, NL, SE)

1. [1,2]-Anellierte 1,4-Benzodiazepin-Verbindungen der allgemeinen Formel I

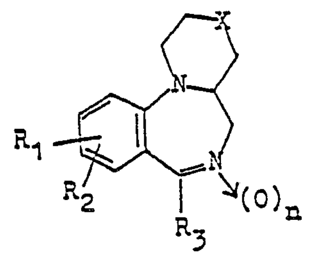

(I)

worin

X für Sauerstoff, Schwefel oder eine Iminogruppe $=N-R_4$ steht, worin

$R_4$ Wasserstoff, $C_1$-$C_5$-Alkyl, endständig durch Methoxy oder Hydroxy substituiertes $C_2$-$C_5$-Alkyl, $C_3$-$C_5$-Alkenyl oder Cyclopropylmethyl bedeutet ;

$R_1$ Wasserstoff, Alkyl mit 1-4 Kohlenstoffatomen, Halogen, Alkoxy mit 1-4 Kohlenstoffatomen oder Nitro bedeutet, und

$R_2$ Wasserstoff, Alkyl mit 1-4 Kohlenstoffatomen, Halogen, Alkoxy mit 1-4 Kohlenstoffatomen oder Nitro bedeutet, oder, falls $R_1$ Wasserstoff ist, auch Alkylthio mit 1-4 Kohlenstoffatomen oder, sofern außerdem X S oder eine $=N-R_4$-Gruppe ist, auch Trifluormethyl bedeutet ; oder

$R_1$ und $R_2$ an benachbarte Kohlenstoffatome gebunden sind und zusammen Methylendioxy oder Äthylendioxy bedeuten ;

$R_3$ für eine der folgenden Gruppen a, b oder c steht

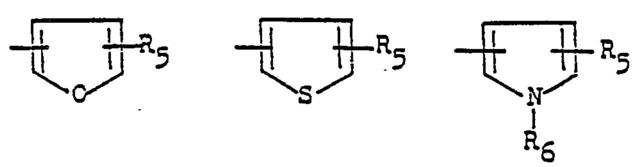

a b c.

worin

$R_5$ Wasserstoff, Alkyl mit 1-4-Kohlenstoffatomen, Fluor, Chlor, Brom oder Nitro bedeutet, und

$R_6$ Wasserstoff, $C_1$-$C_5$-Alkyl, endständig durch Hydroxy oder Methoxy substituiertes $C_2$-$C_5$-Alkyl, $C_3$-$C_5$-Alkenyl oder Cyclopropylmethyl bedeutet ; und

n 0 oder, falls $R_3$ für die Gruppe a oder b steht, auch 1 bedeutet ;

und deren optische Isomere und Saüreadditionssalze.

2. Verbindungen gemäß Anspruch 1, worin $R_1$, $R_2$, $R_3$ und n obige Bedeutung besitzen und X für die Gruppe $=N-R_4$, worin $R_4$ obige Bedeutung besitzt, steht.

3. Verbindungen gemäß Anspruch 2, worin $R_1$, $R_2$, X und n obige Bedeutung besitzen und $R_3$ für die Gruppe a steht.

4. Verbindungen gemäß Anspruch 2, worin $R_1$, $R_2$, X und n obige Bedeutung besitzen und $R_3$ für die Gruppe b steht.

5. Verbindungen gemäß Anspruch 4, worin $R_3$ 2-Thienyl bedeutet, $R_1$ in 9- oder 10-Stellung angeordnet ist und Wasserstoff, Alkyl mit 1-2 Kohlenstoffatomen, Fluor, Chlor oder Methoxy bedeutet, $R_4$ $C_1$-$C_5$-Alkyl bedeutet und n 0 bedeutet.

6. Verfahren zur Herstellung von [1,2]-anellierten 1,4-Benzodiazepin-Verbindungen der allgemeinen Formel I

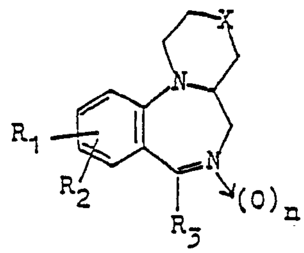

(I)

worin X, $R_1$, $R_2$, $R_3$ und n die in Anspruch 1 angegebene Bedeutung besitzen, und deren optischen Isomeren und Säureadditionssalzen, dadurch gekennzeichnet, daß man

a) Verbindungen der Formel II oder III

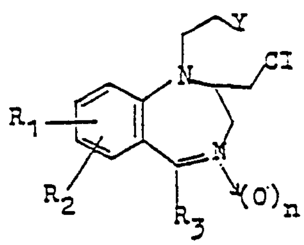

(II)

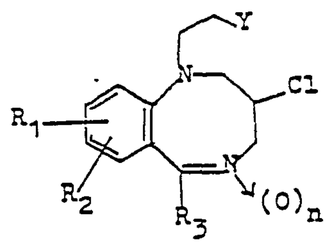

(III)

worin $R_1$, $R_2$, $R_3$ und n obige Bedeutung besitzen und Y Halogen, Alkansulfonyloxy mit 1-4 Kohlenstoffatomen, Benzolsulfonyloxy oder im Benzolring durch Alkyl mit 1-4 Kohlenstoffatomen oder Halogen substituiertes Benzolsulfonyloxy bedeutet, oder deren Gemische mit einem Alkalimetallhydroxyd, einem Alkalimetallsulfid oder einem Amin der Formel $R_4$—$NH_2$, worin $R_4$ obige Bedeutung besitzt, umsetzt, oder

b) zur Herstellung von Verbindungen der Formel Ia

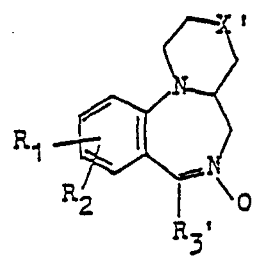

(Ia)

worin $R_1$ und $R_2$ obige Bedeutung besitzen, X' Sauerstoff oder Schwefel bedeutet, und $R_3'$ für eine Gruppe a oder b steht,

Verbindungen der Formel Ib

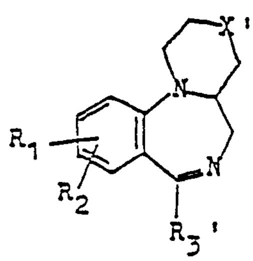

(Ib)

worin $R_1$, $R_2$, $R_3'$ und X' obige Bedeutung besitzen, oxidiert, oder

c) zur Herstellung von Verbindungen der Formel Ic

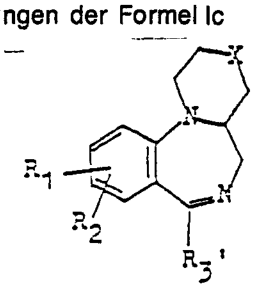

(Ic)

worin $R_1$, $R_2$, $R_3'$ und X obige Bedeutung besitzen,
Verbindungen der Formel Id

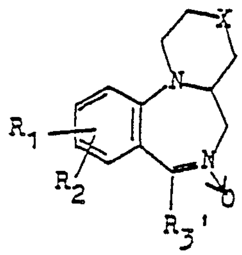

(Id)

worin $R_1$, $R_2$, $R_3'$ und X obige Bedeutung besitzen, reduziert, oder

d) zur Herstellung von Verbindungen der Formel Ie.

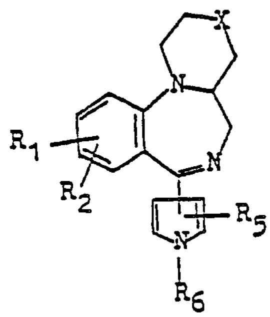

(Ie)

worin $R_1$, $R_2$, $R_5$, $R_6$ und X obige Bedeutung besitzen,
Verbindungen der Formel IV

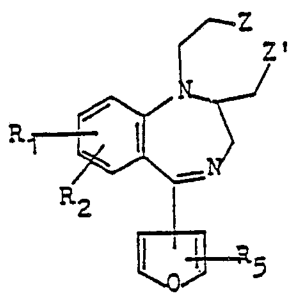

(IV)

worin $R_1$, $R_2$ und $R_5$ obige Bedeutung besitzen, und Z die für Y angegebene Bedeutung besitzt und Z' Chlor bedeutet oder Z und Z' gemeinsam Sauerstoff, Schwefel oder eine Gruppe $=N-R_4$ bedeuten, mit einem Amin der Formel $R_6-NH_2$, worin $R_6$ obige Bedeutung besitzt, umsetzt, und gegebenenfalls in Verbindungen der Formel I, worin $R_1$ und/oder $R_2$ Wasserstoff bedeuten, in den Phenylring Chlor, Brom oder, falls $R_3$ nicht für die Gruppe c steht, auch Nitro einführt, und/oder gegebenenfalls racemische Gemische von Verbindungen der Formel I in ihre optischen Isomeren auftrennt und/oder gegebenenfalls freie Verbindungen der Formel I in ihre Säureadditionssalze überführt oder die Säureadditionssalze in die freien Verbindungen der Formel I überführt.

7. Arzneimittel, enthaltend eine Verbindung gemäß Anspruch 1 und übliche pharmazeutische Trägerstoffe.

8. Verbindungen der Formel II oder III

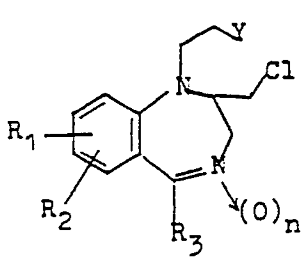

(II)

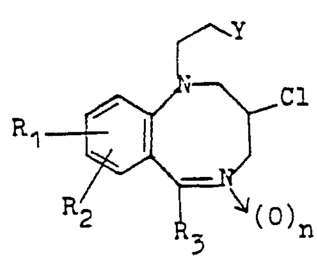

(III)

worin

$R_1$ Wasserstoff, Alkyl mit 1-4 Kohlenstoffatomen, Halogen, Alkoxy mit 1-4 Kohlenstoffatomen oder Nitro bedeutet, und

$R_2$ Wasserstoff, Alkyl mit 1-4 Kohlenstoffatomen, Halogen, Alkoxy mit 1-4 Kohlenstoffatomen oder auch Nitro bedeutet, oder, falls $R_1$ Wasserstoff ist, auch Alkylthio mit 1-4 Kohlenstoffatomen oder Trifluormethyl bedeutet ; oder

$R_1$ und $R_2$ an benachbarte Kohlenstoffatome gebunden sind und zusammen Methylendioxy oder Äthylendioxy bedeuten ;

$R_3$ für eine der folgenden Gruppen a, b oder c steht

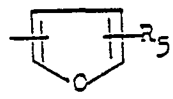 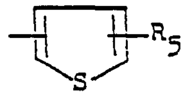 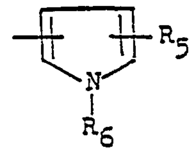

a b c

worin

$R_5$ Wasserstoff, Alkyl mit 1-4 Kohlenstoffatomen, Fluor, Chlor, Brom oder Nitro bedeutet, und

$R_6$ Wasserstoff, $C_1$-$C_5$-Alkyl, endständig durch Hydroxy oder Methoxy substituiertes $C_2$-$C_5$-Alkyl, $C_3$-$C_5$-Alkenyl oder Cyclopropylmethyl bedeutet ;

n 0 oder, falls $R_3$ für die Gruppe a oder b steht, auch 1 bedeutet ; und

Y Halogen, Alkansulfonyloxy mit 1-4 Kohlenstoffatomen, Benzolsulfonyloxy oder im Benzolring durch Alkyl mit 1-4 Kohlenstoffatomen oder Halogen substituiertes Benzolsulfonyloxy bedeutet.

**Patentansprüche** (für den Vertragsstaat AT)

1. Verfahren zur Herstellung von [1,2]-anellierten 1,4-Benzodiazepin-Verbindungen der allgemeinen Formel I

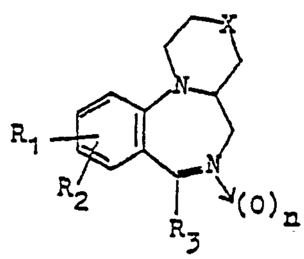

(I)

worin

X für Sauerstoff, Schwefel oder eine Iminogruppe =N—$R_4$ steht, worin

$R_4$ Wasserstoff, $C_1$-$C_5$-Alkyl, endständig durch Methoxy oder Hydroxy substituiertes $C_2$-$C_5$-Alkyl, $C_3$-$C_5$-Alkenyl oder Cyclopropylmethyl bedeutet ;

$R_1$ Wasserstoff, Alkyl mit 1-4 Kohlenstoffatomen, Halogen, Alkoxy mit 1-4 Kohlenstoffatomen oder Nitro bedeutet, und

$R_2$ Wasserstoff, Alkyl mit 1-4 Kohlenstoffatomen, Halogen, Alkoxy mit 1-4 Kohlenstoffatomen oder Nitro bedeutet, oder, falls $R_1$ Wasserstoff ist, auch Alkylthio mit 1-4 Kohlenstoffatomen oder, sofern außerdem X S oder eine =N—$R_4$-Gruppe ist, auch Trifluormethyl bedeutet ; oder

$R_1$ und $R_2$ an benachbarte Kohlenstoffatome gebunden sind und zusammen Methylendioxy oder Äthylendioxy bedeuten ;

$R_3$ für eine der folgenden Gruppen a, b oder c steht

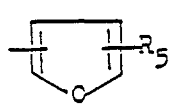 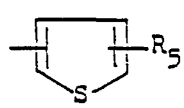 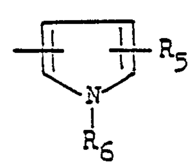

a b c

worin

$R_5$ Wasserstoff, Alkyl mit 1-4 Kohlenstoffatomen, Fluor, Chlor, Brom oder Nitro bedeutet, und

$R_6$ Wasserstoff, $C_1$-$C_5$-Alkyl, endständig durch Hydroxy oder Methoxy substituiertes $C_2$-$C_5$-Alkyl, $C_3$-$C_5$-Alkenyl oder Cyclopropylmethyl bedeutet ; und

n 0 oder, falls $R_3$ für die Gruppe a oder b steht, auch 1 bedeutet ;

und deren optischen Isomeren und Säureadditionssalzen, dadurch gekennzeichnet, daß man

a) Verbindungen der Formel II oder III

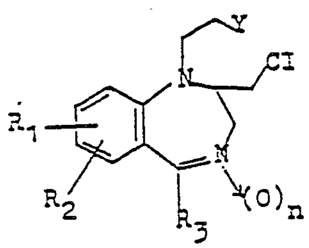

(II)

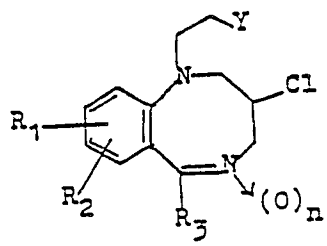

(III)

worin $R_1$, $R_2$, $R_3$ und n obige Bedeutung besitzen und Y Halogen, Alkansulfonyloxy mit 1-4 Kohlenstoffatomen, Benzolsulfonyloxy oder im Benzolring durch niederes Alkyl mit 1-4 Kohlenstoffatomen oder Halogen substituiertes Benzolsulfonyloxy bedeutet, oder deren Gemische mit einem Alkalimetallhydroxyd, einem Alkalimetallsulfid oder einem Amin der Formel $R_4$—$NH_2$, worin $R_4$ obige Bedeutung besitzt, umsetzt, oder

b) zur Herstellung von Verbindungen der Formel Ia

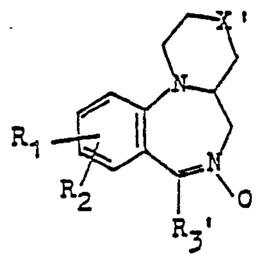

(Ia)

worin $R_1$ und $R_2$ obige Bedeutung besitzen, X' Sauerstoff oder Schwefel bedeutet, und $R_3'$ für eine Gruppe a oder b steht,

Verbindungen der Formel Ib

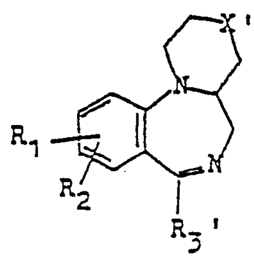

(Ib)

worin $R_1$, $R_2$, $R_3'$ und X' obige Bedeutung besitzen, oxidiert, oder

c) zur Herstellung von Verbindungen der Formel Ic

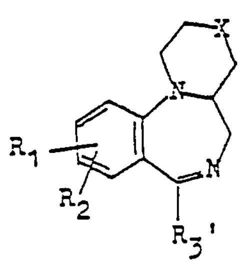

(Ic)

worin $R_1$, $R_2$, $R_3'$ und X obige Bedeutung besitzen,
Verbindungen der Formel Id

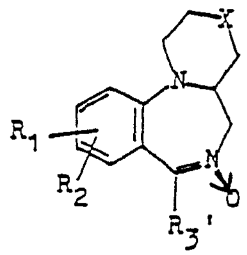

(Id)

worin $R_1$, $R_2$, $R_3'$ und X obige Bedeutung besitzen, reduziert, oder

d) zur Herstellung von Verbindungen der Formel Ie

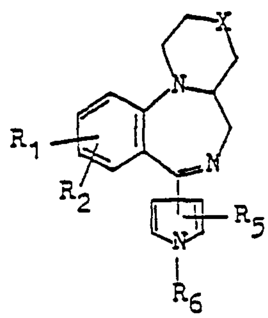

(Ie)

worin $R_1$, $R_2$, $R_5$, $R_6$ und X obige Bedeutung besitzen,
Verbindungen der Formel IV

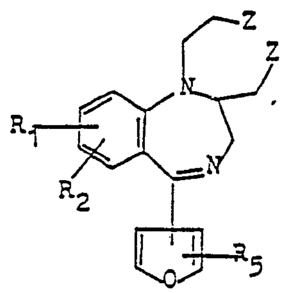

(IV)

worin $R_1$, $R_2$ und $R_5$ obige Bedeutung besitzen, und Z die für Y angegebene Bedeutung besitzt und Z' Chlor bedeutet oder Z und Z' gemeinsam Sauerstoff, Schwefel oder eine Gruppe $=N-R_4$ bedeuten, mit einem Amin der Formel $R_6-NH_2$, worin $R_6$ obige Bedeutung besitzt, umsetzt, und gegebenenfalls in Verbindungen der Formel I, worin $R_1$ und/oder $R_2$ Wasserstoff bedeuten, in den Phenylring Chlor, Brom oder, falls $R_3$ nicht für die Gruppe c steht, auch Nitro einführt, und/oder gegebenenfalls racemische Gemische von Verbindungen der Formel I in ihre optischen Isomeren auftrennt und/oder gegebenenfalls freie Verbindungen der Formel I in ihre Säureadditionssalze überführt oder die Säureadditionssalze in die freien Verbindungen der Formel I überführt.

2. Verfahren gemäß Anspruch 1, dadurch gekennzeichnet, daß man [1,2]-anellierte 1,4-Benzodiazepin-Verbindungen der Formel I herstellt, worin $R_1$, $R_2$, $R_3$ und n obige Bedeutung besitzen und X für die Gruppe $=N-R_4$, worin $R_4$ obige Bedeutung besitzt, steht.

3. Verfahren gemäß Anspruch 2, dadurch gekennzeichnet, daß man [1,2]-anellierte 1,4-Benzodiazepin-Verbindungen der Formel I herstellt, worin $R_1$, $R_2$, X und n obige Bedeutung besitzen und $R_3$ für die Gruppe a steht.

4. Verfahren gemäß Anspruch 2, dadurch gekennzeichnet, daß man [1,2]-anellierte 1,4-Benzodiazepin-Verbindungen der Formel I herstellt, worin $R_1$, $R_2$, X und n obige Bedeutung besitzen und $R_3$ für die Gruppe b steht.

5. Verfahren gemäß Anspruch 4, dadurch gekennzeichnet, daß man [1,2]-anellierte 1,4-Benzodiazepin-Verbindungen der Formel I herstellt, worin $R_3$ 2-Thienyl bedeutet, $R_1$ in 9- oder 10-Stellung angeordnet ist und Wasserstoff, Alkyl mit 1-2 Kohlenstoffatomen, Fluor, Chlor oder Methoxy bedeutet, $R_4$ $C_1$-$C_5$-Alkyl bedeutet und n 0 bedeutet.

6. Verfahren zur Herstellung von Verbindungen der Formel II oder III

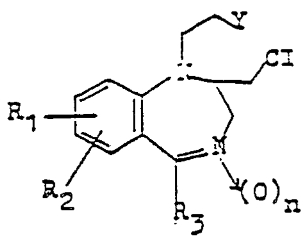

(II)

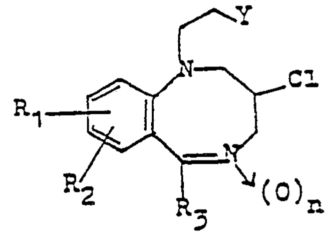

(III)

worin

$R_1$ Wasserstoff, Alkyl mit 1-4 Kohlenstoffatomen, Halogen, Alkoxy mit 1-4 Kohlenstoffatomen oder Nitro bedeutet, und

$R_2$ Wasserstoff, Alkyl mit 1-4 Kohlenstoffatomen, Halogen, Alkoxy mit 1-4 Kohlenstoffatomen oder auch Nitro bedeutet, oder, falls $R_1$ Wasserstoff ist, auch Alkylthio mit 1-4 Kohlenstoffatomen oder Trifluormethyl bedeutet ; oder

$R_1$ und $R_2$ an benachbarte Kohlenstoffatome gebunden sind und zusammen Methylendioxy oder Äthylendioxy bedeuten ;

$R_3$ für eine der folgenden Gruppen a, b oder c steht

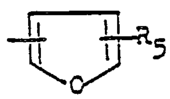

a

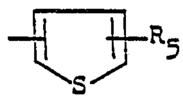

b

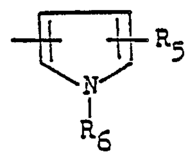

c

worin

$R_5$ Wasserstoff, Alkyl mit 1-4 Kohlenstoffatomen, Fluor, Chlor, Brom oder Nitro bedeutet, und

$R_6$ Wasserstoff, $C_1$-$C_5$-Alkyl, endständig durch Hydroxy oder Methoxy substituiertes $C_2$-$C_5$-Alkyl, $C_3$-$C_5$-Alkenyl oder Cyclopropylmethyl bedeutet ;

n 0 oder, falls $R_3$ für die Gruppe a oder b steht, auch 1 bedeutet ; und

Y Halogen, Alkansulfonyloxy mit 1-4 Kohlenstoffatomen, Benzolsulfonyloxy oder im Benzolring durch Alkyl mit 1-4 Kohlenstoffatomen oder Halogen substituiertes Benzolsulfonyloxy bedeutet, dadurch gekennzeichnet, daß man Verbindungen der allgemeinen Formel VII

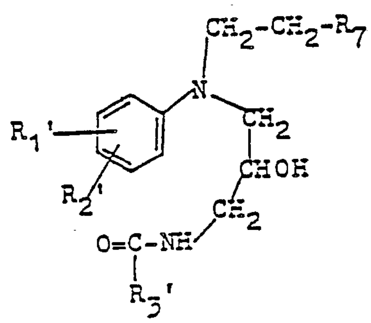

(VII)

worin $R_1'$ und $R_2'$ die für $R_1$ und $R_2$ angegebene Bedeutung mit Ausnahme von Nitro besitzen, $R_3'$ für eine Gruppe a oder b steht und $R_7$ Hydroxy oder Methoxy bedeutet, durch Umsetzung mit Phosphoroxidhalogeniden cyclisiert zu einem Gemisch der beiden isomeren Verbindungen der Formeln VIII und IX

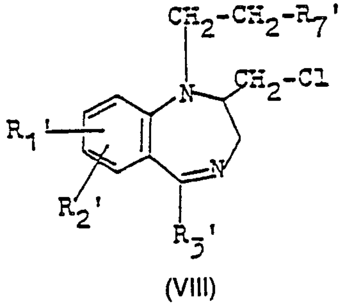

(VIII)

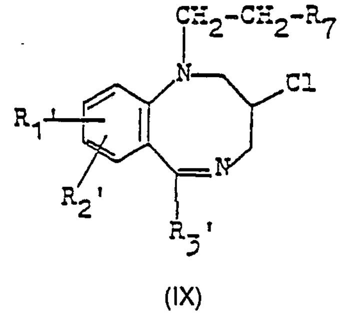

(IX)

worin
$R_1'$, $R_2'$ und $R_3'$ obige Bedeutung besitzen und
$R_7'$ Chlor oder Methoxy bedeutet,

und eine Methoxygruppe $R_7'$ zur Hydroxygruppe spaltet und diese anschließend zu einer Sulfonylestergruppe Y verestert oder in eine Halogengruppe Y überführt und gewünschtenfalls Nitrogruppen $R_1$ oder $R_2$ in den Phenylring einführt und/oder gewünschtenfalls die Verbindungen zu den entsprechenden N-oxiden oxidiert und/oder gewünschtenfalls Verbindungen, worin $R_3'$ Furyl bedeutet, auf an sich bekannte Weise in Verbindungen, worin $R_3$ Pyrrolyl darstellt, überführt, und/oder gewünschtenfalls das Gemisch in seine Isomeren auftrennt.

**Claims** (for the Contracting States : BE, CH, DE, FR, GB, IT, LI, LU, NL, SE)

1. [1,2]-Fused 1,4-benzodiazepine compounds of the general Formula I

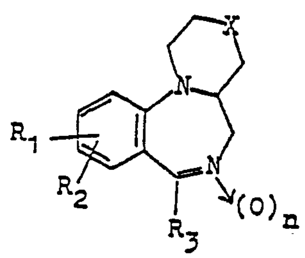

(I)

in which
X stands for oxygen, sulphur or an imino group $=N—R_4$, in which,
$R_4$ is hydrogen, $C_1$-$C_5$-alkyl, $C_2$-$C_5$-alkyl substituted in terminal position by methoxy or hydroxy, $C_3$-$C_5$-alkenyl or cyclopropylmethyl ;
$R_1$ is hydrogen, alkyl with 1 to 4 carbon atoms, halogen, alkoxy with 1 to 4 carbon atoms or nitro, and
$R_2$ is hydrogen, alkyl with 1 to 4 carbon atoms, halogen, alkoxy with 1 to 4 carbon atoms or nitro, or, if $R_1$ is hydrogen, is also alkylthio with 1 to 4 carbon atoms or, in so far as in addition X is S or a $=N—R_4$ group, is also trifluoromethyl ; or
$R_1$ and $R_2$ are linked to adjacent carbon atoms and together denote methylene dioxy or ethylene dioxy ;
$R_3$ stands for one of the following groups a, b or c

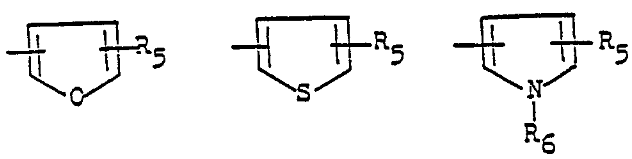

a b c

in which
$R_5$ is hydrogen, alkyl with 1 to 4 carbon atoms, fluorine, chlorine, bromine or nitro, and
$R_6$ is hydrogen, $C_1$-$C_5$-alkyl, $C_2$-$C_5$-alkyl substituted in the terminal position by hydroxy or methoxy, $C_3$-$C_5$-alkenyl or cyclopropylmethyl ; and
n is 0 or, if $R_3$ stands for the group a or b, is also 1 ;
and their optical isomers and acid addition salts.

2. Compounds according to Claim 1, in which $R_1$, $R_2$, $R_3$ and n have the above meaning and X stands for the group $=N—R_4$, in which $R_4$ has the above meaning.

3. Compounds according to Claim 2, in which $R_1$, $R_2$, X and n have the above meaning and $R_3$ stands for the group a.

4. Compounds according to Claim 2, in which $R_1$, $R_2$, X and n have the above meaning and $R_3$ stands for the group b.

5. Compounds according to Claim 4, in which $R_3$ is 2-thienyl, $R_1$ is arranged in position 9 or 10 and is hydrogen, alkyl with 1 or 2 carbon atoms, fluorine, chlorine or methoxy, $R_4$ is $C_1$-$C_5$-alkyl and n is 0.

6. Method for the preparation of [1,2]-fused 1,4-benzodiazepine compounds of the general Formula I

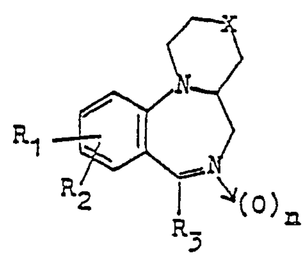

(I)

in which X, $R_1$, $R_2$, $R_3$ and n have the meaning indicated in Claim 1, and their optical isomers and acid addition salts, characterised in that

a) compounds of Formula II or III

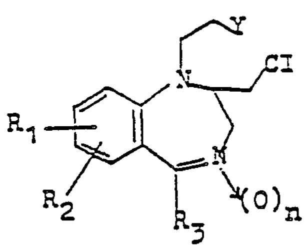

(II)

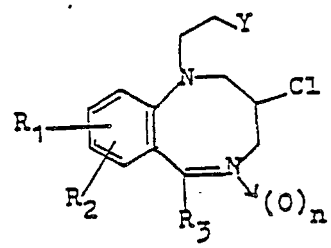

(III)

in which $R_1$, $R_2$, $R_3$ and n have the above meaning and Y is halogen, alkane sulphonyloxy with 1 to 4 carbon atoms, benzene sulphonyloxy or benzene sulphonyloxy substituted in the benzene ring by alkyl with 1 to 4 carbon atoms or by halogen, or mixtures thereof are reacted with an alkali metal hydroxide, an alkali metal sulphide or an amine of the formula $R_4$—$NH_2$, in which $R_4$ has the above meaning, or

b) for the preparation of compounds of Formula Ia

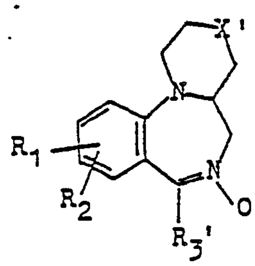

(Ia)

in which $R_1$ and $R_2$ have the above meaning, X′ is oxygen or sulphur, and $R_3'$ stands for a group a or b, compounds of Formula Ib

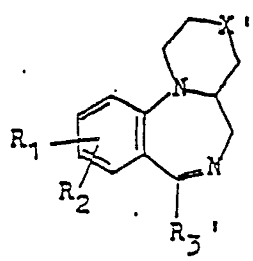

(Ib)

in which $R_1$, $R_2$ and $R_3'$ and X′ have the above meaning are oxidized, or

c) for the preparation of compounds of Formula Ic

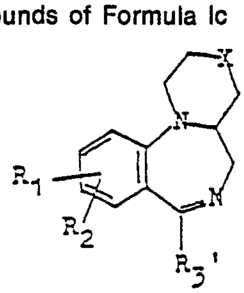

(Ic)

in which $R_1$, $R_2$, $R_3'$ and X have the above meaning, compounds of Formula Id

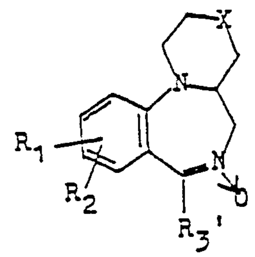

(Id)

in which $R_1$, $R_2$, $R_3'$ and X have the above meaning, are reduced, or

d) for the preparation of compounds of Formula Ie

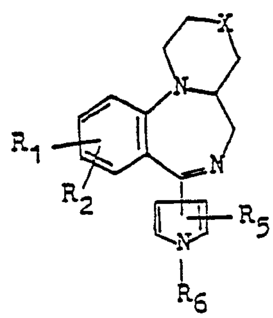

(Ie)

in which $R_1$, $R_2$, $R_5$, $R_6$ and X have the above meaning, compounds of Formula IV

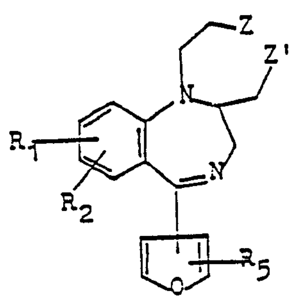

(IV)

in which $R_1$, $R_2$ and $R_5$ have the above meaning, and Z has the meaning indicated for Y and Z' is chlorine or Z and Z' together denote oxygen, sulphur or a group $=N-R_4$, are reacted with an amine of the formula $R_6-NH_2$, in which $R_6$ has the above meaning, and if required in compounds of Formula I, in which $R_1$ and/or $R_2$ is hydrogen, chlorine, bromine or, if $R_3$ does not stand for the group c, also nitro are introduced into the phenyl ring, and/or if required, racemic mixtures of compounds of Formula I are separated into their optical isomers and/of if required free compounds of Formula I are converted into their acid addition salts or the acid addition salts are converted into the free compounds of Formula I.

7. Medicaments containing a compound according to Claim 1 and conventional pharmaceutical carrier substances.

8. Compounds of Formula II or III

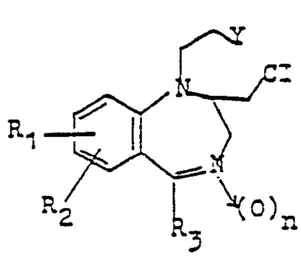

(II)

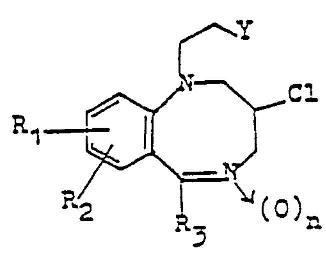

(III)

in which

$R_1$ is hydrogen, alkyl with 1 to 4 carbon atoms, halogen, alkoxy with 1 to 4 carbon atoms or nitro, and

$R_2$ is hydrogen, alkyl with 1 to 4 carbon atoms, halogen, alkoxy with 1 to 4 carbon atoms or also nitro, or, if $R_1$ is hydrogen, is also alkylthio with 1 to 4 carbon atoms or trifluoromethyl ; or

$R_1$ and $R_2$ are linked to adjacent carbon atoms and together denote methylene dioxy or ethylene dioxy ;

$R_3$ stands for one of the following groups a, b or c

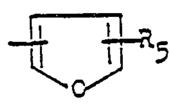 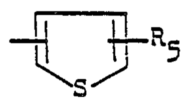 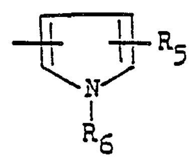

a b c

in which

$R_5$ is hydrogen, alkyl with 1 to 4 carbon atoms, fluorine, chlorine, bromine or nitro, and

$R_6$ is hydrogen, $C_1$-$C_5$-alkyl, $C_2$-$C_5$-alkyl substituted in the terminal position by hydroxy or methoxy, $C_3$-$C_5$-alkenyl or cyclopropylmethyl ;

n is 0 or, if $R_3$ stands for the group a or b, is also 1 ; and

Y is halogen, alkane sulphonyloxy with 1 to 4 carbon atoms, benzene sulphonyloxy or benzene sulphonyloxy substituted in the benzene ring by alkyl with 1 to 4 carbon atoms or by halogen.

**Claims** (for the Contracting State AT)

1. Method for the preparation of [1,2]-fused 1,4-benzodiazepine compounds of the general Formula I

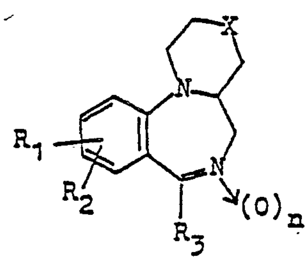

(I)

in which

X stands for oxygen, sulphur or an imino group =N—$R_4$, in which,

$R_4$ is hydrogen, $C_1$-$C_5$-alkyl, $C_2$-$C_5$-alkyl substituted in terminal position by methoxy or hydroxy, $C_3$-$C_5$-alkenyl or cyclopropylmethyl ;

$R_1$ is hydrogen, alkyl with 1 to 4 carbon atoms, halogen, alkoxy with 1 to 4 carbon atoms or nitro, and

$R_2$ is hydrogen, alkyl with 1 to 4 carbon atoms, halogen, alkoxy with 1 to 4 carbon atoms or nitro, or, if $R_1$ is hydrogen, also alkylthio with 1 to 4 carbon atoms or, in so far as in addition X is S or a =N—$R_4$ group, is also trifluoromethyl ; or

$R_1$ and $R_2$ are linked to adjacent carbon atoms and together denote methylene dioxy or ethylene dioxy ;

$R_3$ stands for one of the following groups a, b or c

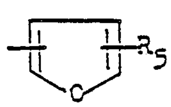 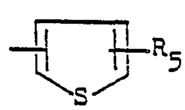 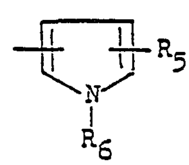

a b c

in which

$R_5$ is hydrogen, alkyl with 1 to 4 carbon atoms, fluorine, chlorine, bromine or nitro, and

$R_6$ is hydrogen, $C_1$-$C_5$-alkyl, $C_2$-$C_5$-alkyl substituted in the terminal position by hydroxy or methoxy, $C_3$-$C_5$-alkenyl or cyclopropylmethyl ; and

n is 0 or, if $R_3$ stands for the group a or b, is also 1 ;

and their optical isomers and acid addition salts, characterised in that

a) compounds of Formula II or III

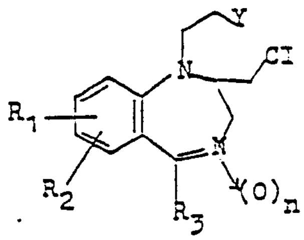

(II)

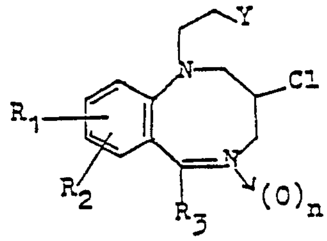

(III)

in which $R_1$, $R_2$, $R_3$ and n have the above meaning and Y is halogen, alkane sulphonyloxy with 1 to 4 carbon atoms, benzene sulphonyloxy or benzene sulphonyloxy substituted in the benzene ring by lower alkyl with 1 to 4 carbon atoms or by halogen, or mixtures thereof are reacted with an alkali metal hydroxide, an alkali metal sulphide or an amine of the formula $R_4$—$NH_2$, in which $R_4$ has the above meaning, or

b) for the preparation of compounds of Formula Ia

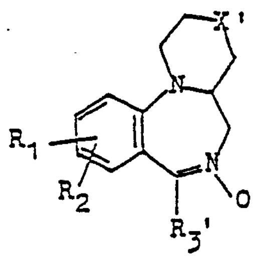

(Ia)

in which $R_1$ and $R_2$ have the above meaning, X' is oxygen or sulphur, and $R_3'$ stands for a group a or b,
compounds of Formula Ib

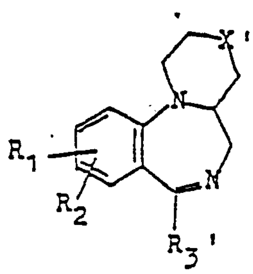

(Ib)

in which $R_1$, $R_2$ and $R_3'$ and X' have the above meaning are oxidized, or

c) for the preparation of compounds of Formula Ic

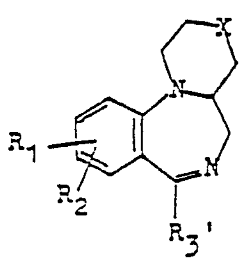

(Ic)

in which $R_1$, $R_2$, $R_3'$ and X have the above meaning,
compounds of Formula Id

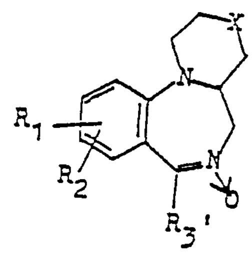

(Id)

in which $R_1$, $R_2$, $R_3'$ and X have the above meaning, are reduced, or

d) for the preparation of compounds of Formula Ie

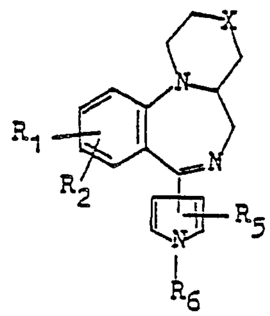

(Ie)

in which $R_1$, $R_2$, $R_5$, $R_6$ and X have the above meaning, compounds of Formula IV

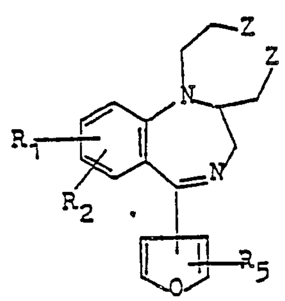

(IV)

in which $R_1$, $R_2$ and $R_5$ have the above meaning, and Z has the meaning indicated for Y and Z' is chlorine or Z and Z' together denote oxygen, sulphur or a group $=N-R_4$, are reacted with an amine of the formula $R_6-NH_2$, in which $R_6$ has the above meaning, and if required in compounds of Formula I, in which $R_1$ and/or $R_2$ is hydrogen, chlorine, bromine or, if $R_3$ does not stand for the group c, also nitro are introduced into the phenyl ring, and/or if required, racemic mixtures of compounds of Formula I are separated into their optical isomers and/or if required free compounds of Formula I are converted into their acid addition salts or the acid addition salts are converted into the free compounds of Formula I.

2. Method according to Claim 1, characterised in that [1,2]-fused 1,4-benzodiazepine compounds of Formula I are prepared, in which $R_1$, $R_2$, $R_3$ and n have the above meaning and X stands for the group $=N-R_4$, in which $R_4$ has the above meaning.

3. Method according to Claim 2, characterised in that [1,2]-fused 1,4-benzodiazepine compounds of Formula I are prepared, in which $R_1$, $R_2$, X and n have the above meaning and $R_3$ stands for the group a.

4. Method according to Claim 2, characterised in that [1,2]-fused 1,4-benzodiazepine compounds of Formula I are prepared, in which $R_1$, $R_2$, X and n have the above meaning and $R_3$ stands for the group b.

5. Method according to Claim 4, characterised in that [1,2]-fused 1,4-benzodiazepine compounds of Formula I are prepared, in which $R_3$ is 2-thienyl, $R_1$ is arranged in position 9 or 10 and is hydrogen, alkyl with 1 or 2 carbon atoms, fluorine, chlorine or methoxy, $R_4$ is $C_1$-$C_5$-alkyl and n is 0.

6. Method for the preparation of compounds of Formula II or III

(See Formula page 35)

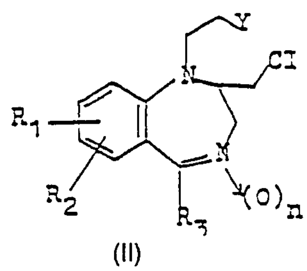

(II)

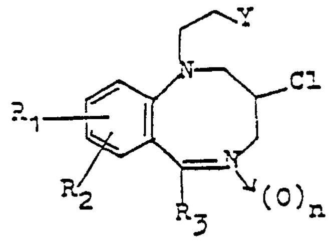

(III)

in which

$R_1$ is hydrogen, alkyl with 1 to 4 carbon atoms, halogen, alkoxy with 1 to 4 carbon atoms or nitro, and

$R_2$ is hydrogen, alkyl with 1 to 4 carbon atoms, halogen, alkoxy with 1 to 4 carbon atoms or also nitro, or, if $R_1$ is hydrogen, is also alkylthio with 1 to 4 carbon atoms or trifluoromethyl ; or

$R_1$ and $R_2$ are linked to adjacent carbon atoms and together denote methylene dioxy or ethylene dioxy ;

$R_3$ stands for one of the following groups a, b or c

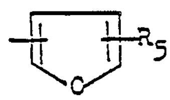

a

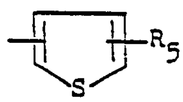

b

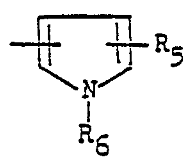

c

in which

$R_5$ is hydrogen, alkyl with 1 to 4 carbon atoms, fluorine, chlorine, bromine or nitro, and

$R_6$ is hydrogen, $C_1$-$C_5$-alkyl, $C_2$-$C_5$-alkyl substituted in the terminal position by hydroxy or methoxy, $C_3$-$C_5$-alkenyl or cyclopropylmethyl ;

n is 0 or, if $R_3$ stands for the group a or b, is also 1 ; and

Y is halogen, alkane sulphonyloxy with 1 to 4 carbon atoms, benzene sulphonyloxy or benzene sulphonyloxy substituted in the benzene ring by alkyl with 1 to 4 carbon atoms or by halogen, characterised in that compounds of the general Formula VII

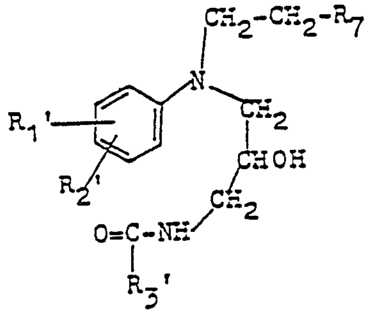

(VII)

in which $R_1'$ and $R_2'$ have the meaning indicated for $R_1$ and $R_2$ with the exception of nitro, $R_3'$ stands for a group a or b and $R_7$ is hydroxy or methoxy, are cyclized by reaction with phosphorus oxide halides to a mixture of the two isomeric compounds of Formulae VIII and IX

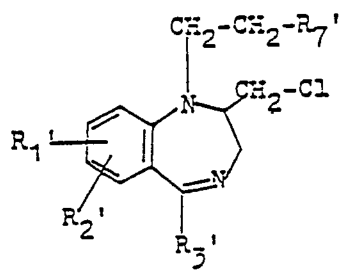

(VIII)

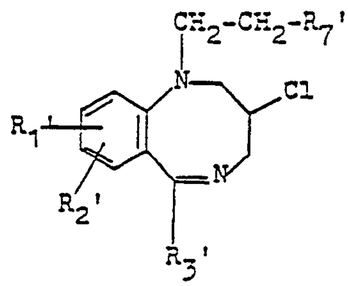

(IX)

in which

$R_1'$, $R_2'$ and $R_3'$ have the above meaning and

$R_7'$ is chlorine or methoxy,

and a methoxy group $R_7'$ is split to the hydroxy group and this is then esterified to a sulphonyl ester group Y or is converted into a halogen group Y and if desired nitro groups $R_1$ and $R_2$ are introduced into the

phenyl ring and/or if desired the compounds are oxidized to the corresponding N-oxides and/or if desired compounds, in which $R_3'$ denotes furyl are converted in a manner known *per se* into compounds in which $R_3$ represents pyrrolyl, and/or if desired the mixture is separated into its isomers.

**Revendications** (pour les Etats contractants : BE, CH, DE, FR, GB, IT, LI, LU, NL, SE)

1. 1,4-benzodiazépines-[1,2] à noyaux condensés, de formule générale I :

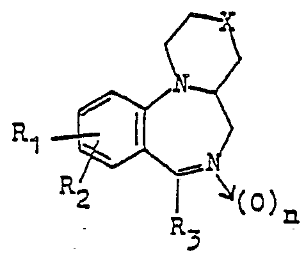

(I)

dans laquelle

X représente un atome d'oxygène ou de soufre ou un groupe imino $=N-R_4$, où

$R_4$ représente un atome d'hydrogène, un groupe alkyle en $C_1$ à $C_5$, un groupe alkyle en $C_2$ à $C_5$ substitué à son extrémité par un groupe méthoxy ou hydroxy, un groupe alcényle en $C_3$ à $C_5$ ou un groupe cyclopropylméthyle ;

$R_1$ représente un atome d'hydrogène, un groupe alkyle ayant 1 à 4 atomes de carbone, un atome d'halogène, un groupe alcoxy ayant 1 à 4 atomes de carbone ou un groupe nitro, et

$R_2$ représente un atome d'hydrogène, un groupe alkyle ayant 1 à 4 atomes de carbone, un atome d'halogène, un groupe alcoxy ayant 1 à 4 atomes de carbone ou un groupe nitro ou, si $R_1$ représente un atome d'hydrogène, $R_2$ peut représenter aussi un groupe alkylthio ayant 1 à 4 atomes de carbone ou dans la mesure où, en outre, X représente S ou un groupe $=N-R_4$, $R_2$ peut représenter aussi un groupe trifluorométhyle ; ou

$R_1$ et $R_2$ sont fixés sur des atomes de carbone voisins et forment ensemble un groupe méthylène dioxy ou éthylènedioxy ;

$R_3$ représente l'un des groupes a, b ou c suivants :

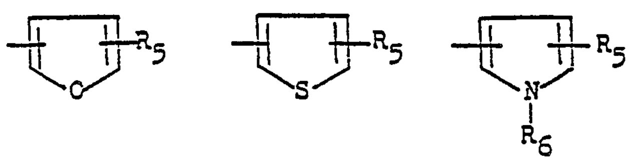

a b c

dans lesquels :

$R_5$ représente un atome d'hydrogène, un groupe alkyle ayant 1 à 4 atomes de carbone, un atome de fluor, de chlore ou de brome ou un groupe nitro, et

$R_6$ représente un atome d'hydrogène, un groupe alkyle en $C_1$ à $C_5$, un groupe alkyle en $C_2$ à $C_5$ substitué à son extrémité par un groupe hydroxy ou méthoxy, un groupe alcényle en $C_3$ à $C_5$ ou un groupe cyclopropylméthyle ; et

n est nul ou, si $R_3$ représente le groupe a ou b, n peut valoir également 1 ;

et leurs isomères optiques et sels d'addition d'acides.

2. Composés selon la revendication 1, dans lesquels $R_1$, $R_2$, $R_3$ et n ont le sens indiqué ci-dessus et X représente le groupe $=N-R_4$, dans lequel $R_4$ a le sens ci-dessus.

3. Composés selon la revendication 2, dans lesquels $R_1$, $R_2$, X et n ont le sens ci-dessus et $R_3$ représente le groupe a.

4. Composés selon la revendication 2, dans lesquels $R_1$, $R_2$, X et n ont le sens ci-dessus et $R_3$ représente le groupe b.

5. Composés selon la revendication 4, dans lesquels $R_3$ représente un groupe 2-thiényle, $R_1$ est en position 9 ou 10 et représente un atome d'hydrogène, un groupe alkyle ayant 1 ou 2 atomes de carbone, un atome de fluor, de chlore ou un groupe méthoxy ; $R_4$ représente un groupe alkyle en $C_1$ à $C_5$ et n est nul.

6. Procédé pour préparer des 1,4-benzodiazépines-[1,2] à noyaux condensés, de formule générale I :

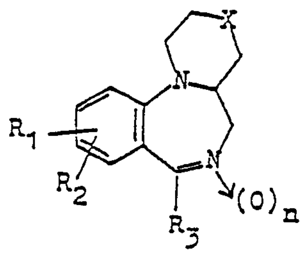

(I)

dans laquelle X, $R_1$, $R_2$, $R_3$ et n ont le sens indiqué à la revendication 1, et leurs isomères optiques et sels d'addition d'acides, procédé caractérisé en ce que :

a) on fait réagir des composés de formule II ou III :

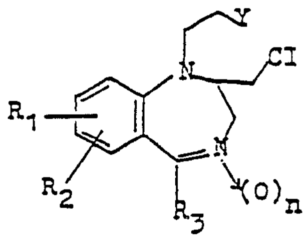

(II)

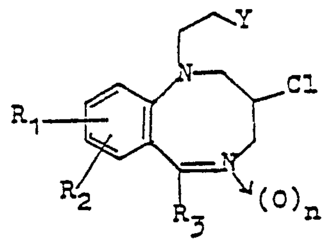

(III)

dans lesquelles $R_1$, $R_2$, $R_3$ et n ont le sens ci-dessus et Y représente un atome d'halogène, un groupe alcane sulfonyloxy ayant 1 à 4 atomes de carbone, benzènesulfonyloxy ou benzènesulfonyloxy substitué dans le noyau benzénique par un groupe alkyle ayant 1 à 4 atomes de carbone ou par de l'halogène, ou leurs mélanges, avec un hydroxyde de métal alcalin, avec un sulfure de métal alcalin ou avec une amine de formule $R_4—NH_2$, dans laquelle $R_4$ a le sens ci-dessus, ou

b) pour préparer des composés de formule Ia :

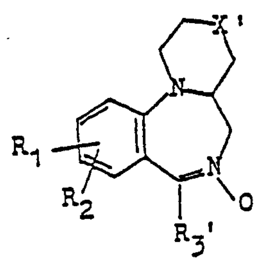

(Ia)

dans laquelle $R_1$ et $R_2$ ont le sens ci-dessus, X' représente un atome d'oxygène ou de soufre et $R_3'$ représente un groupe a ou b,

on oxyde des composés de formule Ib :

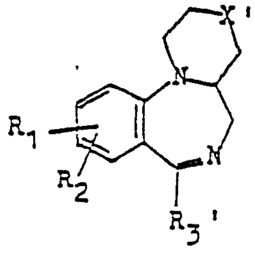

(Ib)

dans laquelle $R_1$, $R_2$, $R_3'$ et X' ont le sens ci-dessus, ou

c) pour préparer des composés de formule Ic :

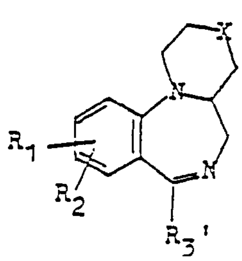

(Ic)

dans laquelle $R_1$, $R_2$, $R_3'$ et X ont le sens ci-dessus,
on réduit des composés de formule Id :

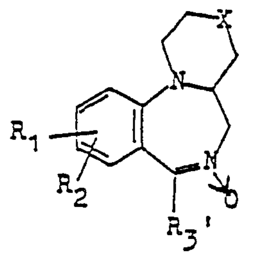

(Id)

dans laquelle $R_1$, $R_2$, $R_3'$ et X ont le sens ci-dessus, ou

d) pour préparer des composés de formule Ie :

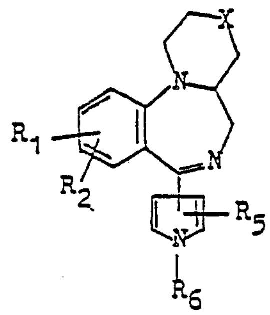

(Ie)

dans laquelle $R_1$, $R_2$, $R_5$, $R_6$ et X ont le sens ci-dessus,
on fait réagir des composés de formule IV :

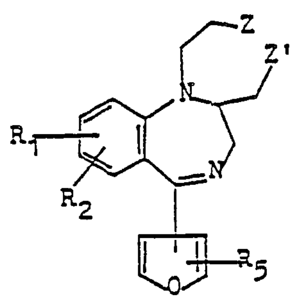

(IV)

dans laquelle $R_1$, $R_2$ et $R_5$ ont le sens ci-dessus, et Z a le sens indiqué pour Y et Z' représente un atome de chlore ou bien Z et Z' représentent ensemble un atome d'oxygène ou de soufre ou un groupe $=N—R_4$, avec une amine de formule $R_6—NH_2$, dans laquelle $R_6$ a le sens ci-dessus, et éventuellement on introduit dans le noyau phényle un atome de chlore ou de brome dans les composés de formule I, dans laquelle $R_1$ et/ou $R_2$ représentent des atomes d'hydrogène, ou, si $R_3$ ne représente pas le groupe c, on introduit également un groupe nitro, et/ou l'on sépare éventuellement les mélanges racémiques de composés de formule I en leurs isomères optiques et/ou on transforme éventuellement les composés libres de formule I en leurs sels d'addition d'acides ou l'on transforme les sels d'addition d'acides en les composés libres de formule I.

7. Médicament, contenant un composé selon la revendication 1 et des excipients pharmaceutiques usuels.

8. Composés de formule II ou III :

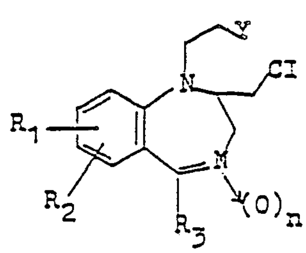

(II)

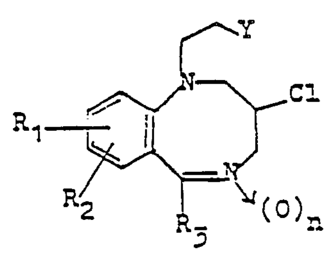

(III)

dans lesquelles :

$R_1$ représente un atome d'hydrogène, un groupe alkyle ayant 1 à 4 atomes de carbone, un atome d'halogène, un groupe alcoxy ayant 1 à 4 atomes de carbone ou un groupe nitro, et

$R_2$ représente un atome d'hydrogène, un groupe alkyle ayant 1 à 4 atomes de carbone, un atome d'halogène, un groupe alcoxy ayant 1 à 4 atomes de carbone ou peut représenter aussi un groupe nitro ou, si $R_1$ représente un atome d'hydrogène, $R_2$ peut représenter aussi un groupe alkylthio ayant 1 à 4 atomes de carbone ou un groupe trifluorométhyle ; ou

$R_1$ et $R_2$ sont fixés sur des atomes de carbone voisins et forment ensemble un groupe méthylènedioxy ou éthylènedioxy ;

$R_3$ représente l'un des groupes a, b ou c suivants :

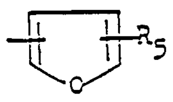
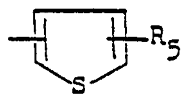
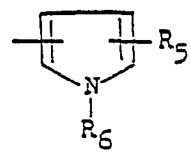

a b c

dans lesquels :

$R_5$ représente un atome d'hydrogène, un groupe alkyle ayant 1 à 4 atomes de carbone, un atome de fluor, de chlore ou de brome ou un groupe nitro, et

$R_6$ représente un atome d'hydrogène, un groupe alkyle en $C_1$ à $C_5$, un groupe alkyle en $C_2$ à $C_5$ substitué à son extrémité par un groupe hydroxy ou méthoxy, un groupe alcényle en $C_3$ à $C_5$ ou un groupe cyclopropylméthyle ;

n est nul ou, si $R_3$ représente le groupe a ou b, n peut aussi valoir 1 ; et

Y représente un atome d'halogène, un groupe alcanesulfonyloxy ayant 1 à 4 atomes de carbone, benzènesulfonyloxy ou un groupe benzènesulfonyloxy substitué dans le noyau benzénique par un groupe alkyle ayant 1 à 4 atomes de carbone ou par un atome d'halogène.

**Revendications** (pour l'Etat contractant AT)

1. Procédé pour préparer des 1,4-benzodiazépines-[1,2] à noyaux condensés, de formule générale I :

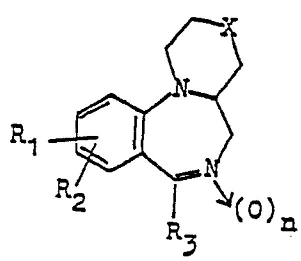

(I)

dans laquelle :

X représente un atome d'oxygène ou de soufre ou un groupe imino $=N-R_4$, où

$R_4$ représente un atome d'hydrogène, un groupe alkyle en $C_1$ à $C_5$, un groupe alkyle en $C_2$ à $C_5$ substitué à son extrémité par un groupe méthoxy ou hydroxy, un groupe alcényle en $C_3$ à $C_5$ ou un groupe cyclopropylméthyle ;

$R_1$ représente un atome d'hydrogène, un groupe alkyle ayant 1 à 4 atomes de carbone, un atome d'halogène, un groupe alcoxy ayant 1 à 4 atomes de carbone ou un groupe nitro, et

$R_2$ représente un atome d'hydrogène, un groupe alkyle ayant 1 à 4 atomes de carbone, un atome d'halogène, un groupe alcoxy ayant 1 à 4 atomes de carbone ou un groupe nitro ou, si $R_1$ représente un atome d'hydrogène, $R_2$ peut représenter aussi un groupe alkylthio ayant 1 à 4 atomes de carbone ou, dans la mesure où en outre X représente S ou un groupe $=N-R_4$, $R_2$ peut représenter aussi un groupe trifluorométhyle ; ou

$R_1$ et $R_2$ sont fixés sur des atomes de carbone voisins et forment ensemble un groupe méthylènedioxy ou éthylènedioxy ;

$R_3$ représente l'un des groupes a, b ou c suivants :

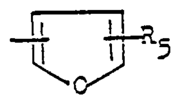 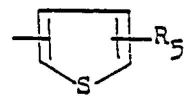 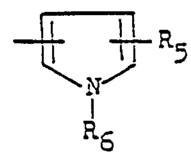

a b c

dans lesquels :

$R_5$ représente un atome d'hydrogène, un groupe alkyle ayant 1 à 4 atomes de carbone, un atome de fluor, de chlore ou de brome ou un groupe nitro, et

$R_6$ représente un atome d'hydrogène, un groupe alkyle en $C_1$ à $C_5$, un groupe alkyle en $C_2$ à $C_5$ substitué à son extrémité par un groupe hydroxy ou méthoxy, un groupe alcényle en $C_3$ à $C_5$ ou un groupe cyclopropylméthyle ; et

n est nul ou, si $R_3$ représente le groupe a ou b, n peut aussi valoir 1 ;

et leurs isomères optiques et sels d'addition d'acides, procédé caractérisé en ce que :

a) on fait réagir des composés de formule II ou III :

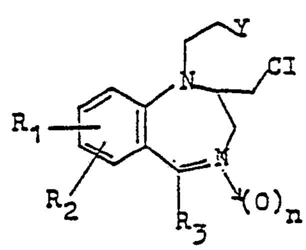

(II)

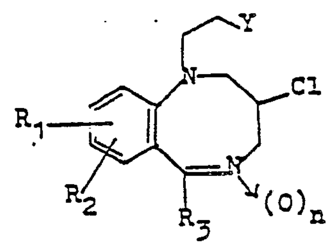

(III)

dans lesquelles $R_1$, $R_2$, $R_3$ et n ont le sens ci-dessus et Y représente un atome d'halogène, un groupe alcane-sulfonyloxy ayant 1 à 4 atomes de carbone, benzènesulfonyloxy ou benzènesulfonyloxy substitué dans le noyau benzénique par un groupe alkyle ayant 1 à 4 atomes de carbone ou par de l'halogène, ou leurs mélanges, avec un hydroxyde de métal alcalin, avec un sulfure de métal alcalin ou avec une amine de formule $R_4—NH_2$, dans laquelle $R_4$ a le sens ci-dessus ; ou

b) pour préparer des composés de formule Ia :

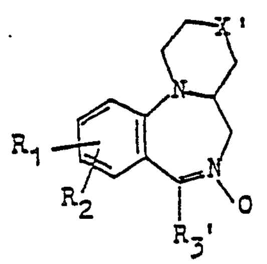

(Ia)

dans laquelle $R_1$ et $R_2$ ont le sens ci-dessus, X' représente un atome d'oxygène ou de soufre et $R_3'$ représente un groupe a ou b,

on oxyde des composés de formule Ib :

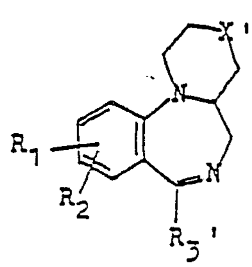

(Ib)

dans laquelle $R_1$, $R_2$, $R_3'$ et X' ont le sens ci-dessus, ou

c) pour préparer des composés de formule Ic :

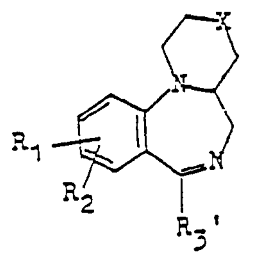

(Ic)

dans laquelle $R_1$, $R_2$, $R_3'$ et X ont le sens ci-dessus,

on réduit des composés de formule Id :

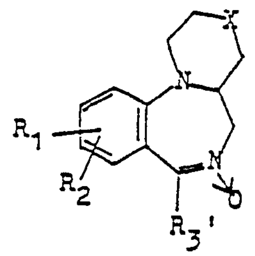

(Id)

dans laquelle $R_1$, $R_2$, $R_3'$ et X ont le sens ci-dessus, ou

d) pour préparer des composés de formule Ie :

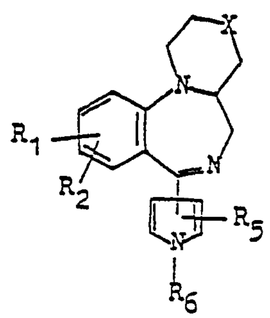

(Ie)

dans laquelle $R_1$, $R_2$, $R_5$, $R_6$ et X ont le sens ci-dessus,

on fait réagir des composés de formule IV :

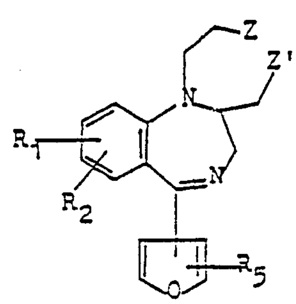

(IV)

dans laquelle $R_1$, $R_2$ et $R_5$ ont le sens ci-dessus et Z a le sens indiqué pour Y et Z' représente un atome de chlore, ou bien Z et Z' représentent ensemble un atome d'oxygène et de soufre ou un groupe $=N—R_4$, avec une amine de formule $R_6—NH_2$, dans laquelle $R_6$ a le sens ci-dessus, et l'on introduit éventuellement dans le noyau phényle les composés de formule I, dans laquelle $R_1$ et/ou $R_2$ représentent chacun un atome d'hydrogène, un atome de chlore ou de brome ou, si $R_3$ ne représente pas le groupe c, on peut introduire également un groupe nitro, et/ou on sépare éventuellement les mélanges racémiques de composés de formule I en leurs isomères optiques et/ou on transforme éventuellement les composés libres de formule I en leurs sels d'addition d'acides ou l'on transforme les sels d'addition d'acides en les composés libres de formule I.

2. Procédé selon la revendication 1, caractérisé en ce qu'on prépare des 1,4-benzodiazépines [1,2] à noyaux condensés de formule I, dans laquelle $R_1$, $R_2$, $R_3$ et n ont le sens ci-dessus et X représente le groupe $=N—R_4$, dans lequel $R_4$ a le sens ci-dessus.

3. Procédé selon la revendication 2, caractérisé en ce qu'on prépare des 1,4-benzodiazépines [1,2] à noyaux condensés de formule I, dans laquelle $R_1$, $R_2$, X et n ont le sens ci-dessus et $R_3$ représente le

groupe a.

4. Procédé selon la revendication 2, caractérisé en ce qu'on prépare des 1,4-benzodiazépines [1,2] à noyaux condensés de formule I, dans laquelle $R_1$, $R_2$, X et n ont le sens ci-dessus et $R_3$ représente le groupe b.

5. Procédé selon la revendication 4, caractérisé en ce qu'on prépare des 1,4-benzodiazépines [1,2] à noyaux condensés de formule I, dans laquelle $R_3$ représente un groupe 2-thiényle, $R_1$ est situé en position 9 ou 10 et représente un atome d'hydrogène, un groupe alkyle ayant 1 ou 2 atomes de carbone, un atome de fluor, de chlore ou un groupe méthoxy, $R_4$ représente un groupe alkyle en $C_1$ à $C_5$ et n est nul.

6. Procédé pour préparer des composés de formule II ou III :

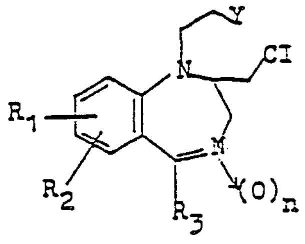
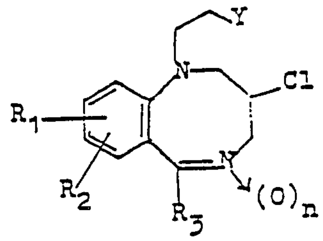

(II) (III)

dans lesquelles :

$R_1$ représente un atome d'hydrogène, un groupe alkyle ayant 1 à 4 atomes de carbone, un atome d'halogène, un groupe alcoxy ayant 1 à 4 atomes de carbone ou un groupe nitro, et

$R_2$ représente un atome d'hydrogène, un groupe alkyle ayant 1 à 4 atomes de carbone, un atome d'halogène, un groupe alcoxy ayant 1 à 4 atomes de carbone ou peut représenter aussi un groupe nitro ou, si $R_1$ représente un atome d'hydrogène, $R_2$ peut représenter aussi un groupe alkylthio ayant 1 à 4 atomes de carbone ou un groupe trifluorométhyle ; ou

$R_1$ et $R_2$ sont fixés sur des atomes de carbone voisins et forment ensemble un groupe méthylènedioxy ou éthylènedioxy ;

$R_3$ représente l'un des groupes a, b ou c suivants :

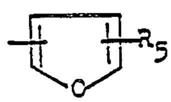
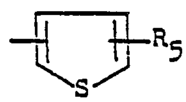
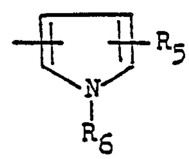

a b c

dans lesquels :

$R_5$ représente un atome d'hydrogène, un groupe alkyle ayant 1 à 4 atomes de carbone, un atome de fluor, de chlore ou de brome ou un groupe nitro, et

$R_6$ représente un atome d'hydrogène, un groupe alkyle ayant 1 à 5 atomes de carbone, un groupe alkyle ayant 2 à 5 atomes de carbone substitué à son extrémité par un groupe hydroxy ou éthoxy, un groupe alcényle en $C_3$ à $C_5$ ou un groupe cyclopropylméthyle ;

n est nul ou, si $R_3$ représente le groupe a ou b, n peut également valoir 1 ; et

Y représente un atome d'halogène, un groupe alcanesulfonyloxy ayant 1 à 4 atomes de carbone, benzènesulfonyloxy ou un groupe benzènesulfonyloxy substitué dans le noyau benzénique par un groupe alkyle ayant 1 à 4 atomes de carbone ou par un atome d'halogène,

procédé caractérisé en ce qu'on cyclise des composés de formule générale VII :

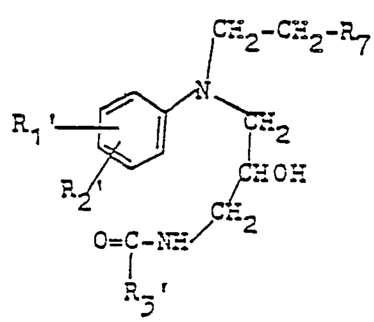

(VII)

dans laquelle $R_1'$ et $R_2'$ ont le sens indiqué pour $R_1$ et $R_2$ à l'exclusion d'un groupe nitro, $R_3'$ représente

un groupe a ou b et $R_7$ représente un groupe hydroxy ou méthoxy, par réaction avec des oxyhalogénures de phosphore pour obtenir un mélange des deux isomères répondant aux formules VIII et IX :

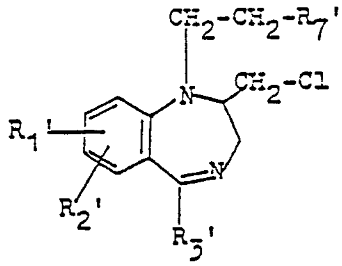

(VIII)

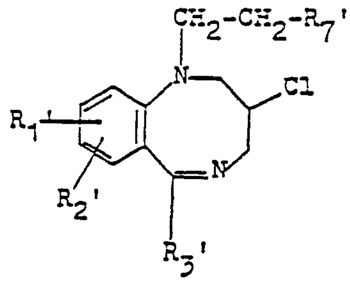

(IX)

dans lesquelles :

$R_1'$, $R_2'$ et $R_3'$ ont le sens ci-dessus, et

$R_7'$ représente un atome de chlore ou un groupe méthoxy,

et l'on scinde un groupe méthoxy $R_7'$ en un groupe hydroxy et l'on estérifie ensuite celui-ci en un groupe ester de sulfonyle Y ou bien on le transforme en un groupe halogéno Y et, éventuellement, on introduit des groupes nitro $R_1$ ou $R_2$ dans le noyau phényle et/ou on oxyde éventuellement les composés pour obtenir les N-oxydes correspondants et/ou on transforme les composés, dans lesquels $R_3'$ représente un groupe furyle, de façon connue en soi en des composés dans lesquels $R_3$ représente un groupe pyrrolyle et/ou on sépare éventuellement le mélange en ses isomères.